(19) Europäisches Patentamt
European Patent Office
Office européen des brevets

(11) EP 2 497 462 A1

(12) # EUROPEAN PATENT APPLICATION

(43) Date of publication:
**12.09.2012 Bulletin 2012/37**

(21) Application number: **12159415.4**

(22) Date of filing: **30.05.2008**

(51) Int Cl.:
***A61K 9/00*** (2006.01) ***A61K 9/08*** (2006.01)
***A61K 9/19*** (2006.01) ***A61K 38/04*** (2006.01)
***A61K 38/16*** (2006.01) ***A61K 47/10*** (2006.01)
***A61K 47/42*** (2006.01) ***A61K 9/12*** (2006.01)
***A61K 38/28*** (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **01.06.2007 EP 07109435**
**17.08.2007 EP 07114524**

(62) Document number(s) of the earlier application(s) in accordance with Art. 76 EPC:
**08760287.6 / 2 164 459**

(71) Applicant: **Novo Nordisk A/S**
**2880 Bagsværd (DK)**

(72) Inventors:
- **Bjerregaard Jensen, Simon**
  **2880 Bagsvaerd (DK)**
- **Havelund, Svend**
  **2880 Bagsvaerd (DK)**
- **Föger, Anders Florian**
  **2880 Bagsvaerd (DK)**
- **Müller, Bernd**
  **24220 Flintbek (DE)**

Remarks:
•This application was filed on 14-03-2012 as a divisional application to the application mentioned under INID code 62.
•Claims filed after the date of receipt of the divisional application (Rule 68(4) EPC).

(54) **Stable non-aqueous pharmaceutical compositions**

(57) The present invention relates to shelf stable non-aqueous pharmaceutical compositions, and to the use thereof in methods of treating diabetes and hyperglycaemia. The composition comprises a dehydrated therapeutically active insulinotropic peptide, and at least one semi-polar protic organic solvent which insulinotropic peptide has been dehydrated at target pH which is at least 1pH unit from the pI of the peptide in aqueous solution.

EP 2 497 462 A1

**Description**

## FIELD OF THE INVENTION

The present invention relates to non-aqueous pharmaceutical compositions, and to the use thereof in methods of treating diabetes and hyperglycaemia.

## BACKGROUND OF INVENTION

Polypeptides have typically low solubilities in most non-aqueous solvents except certain aprotic solvents like dimethyl sulfoxide (DMSO), dimethylacetamide and dimethylformamide. However, the toxicity of these aprotic solvents disqualifies them from being used in pharmaceutical formulations to any significant degree.

Klibanov et al (J. T. Chin, S. L. Wheeler, and A. M. Klibanov. Communication to the editor: On protein solubility in organic solvents. BIOTECHNOL.BIOENG. 44 (1):140-145, 1994) describesthat protic, very hydrophilic and polar solvents dissolve more than 10 mg/ml lysozyme(lyophilized fromaqueous solution of pH 6.0). The solubility in an individual solvent (1,5 pentanediol) markedly increased when the lysozyme was freeze dried prior to dissolution in pentanediol from aqueous solutions where pH values were moved below the isoelectric point of lysozyme. No strong correlation between solvent characteristics and solubility of lysozyme could be established.

WO 00/42993 describes formulations for delivery of macromolecules, where the macromolecules are dissolved or dispersed in a low toxicity organic solvent which can be aerosolized for delivery to a patient's lung by inhalation.

Many organic polar protic solvents tend todestabilize polypeptides such as insulinotropic peptides due to partial unfolding of the polypeptides, which often enhances aggregation and chemical degradation processes significantly due to higher conformational flexibility. Some organic polar protic solvents like ethanol might even act as a denaturant for polypeptides. Furthermore, the organic polar protic solvents often contain small amounts of highly reactive impurities such as aldehydes and ketones, which compromises the stability of the polypeptide. Hence, it is difficult to formulate a non-aqueous solution of aninsulinotropic peptide with adequate shelf life stability.

Because non-aqueous insulinotropic peptide solutions can be further processed into solution pressurized metered dose inhalers (pMDI), where the polar non-aqueous act as a co-solvent in order for the insulinotropic peptide to be solubilized in a hydrofluoroalkane they can be advantageously used for pulmonal administration.

The non-aqueous insulinotropic peptide solutions can also be processed into microemulsions for oral administration by adding detergents and non-polar hydrophobic solvents such as oils. The microemulsion might protect the insulinotropic peptide against proteolytic degradation and enhance the systemic absorption of the insulinotropic peptide from the gastrointestinal tract. Furthermore, it is anticipated that hydrolysis of the insulinotropic peptides is minimized in non-aqueous formulations due to lower water activity.

Various treatments and addition of excipients must often be applied to non-aqueous pharmaceutical compositions of therapeutic peptides in order to improve their solubility and their stability.

Shelf life of liquid parenteral formulations of peptides must be at least a year, preferably longer. The in-use period where the product may be transported and shaken daily at ambient temperature preferably should be several weeks.

Thus, there is a need for non-aqueous pharmaceutical compositions of therapeutic peptides which have improved stability.

## SUMMARY OF THE INVENTION

In one aspect of the invention, a pharmaceutical non-aqueous composition comprising a dehydrated insulinotropic peptide, andat least onesemi-polar protic organic solventwhich insulinotropic peptide has been dehydratedat a target pH which is at least 1 pH unit from the pI of the insulinotropic peptide in aqueous solution, is provided.

In one aspect of the invention, a pharmaceutical non-aqueous composition comprising a dehydrated insulinotropic peptide, andat least onesemi-polar protic organic solventwhich insulinotropic peptide has been dehydratedat a target pH which is at least 1 pH unit from the pI of the insulinotropic peptide in aqueous solution, and where said target pH is in the range from about 6.0 to about 9.0, is provided.

## DETAILED DESCRIPTION OF THE INVENTION

We have discovered that insulinotropic peptides such as GLP-1 in solid state (dehydrated) can be solubilized to a very high degree in a non-aqueous semi-polar protic solvent by optimizing the pH of said insulinotropic peptides in an aqueous solutionbefore dehydration making the formulationof shelf-stable pharmaceutical formulations possible. Such pharmaceutical compositions for e.g. oral, pulmonal and nasal use show high chemical and/or physical stability.

Compositions according to the invention such as oral pharmaceutical compositions using propylene glycol as a semi-polar protic organic solventmay also show significantly improved bioavailability.

In one aspect of the invention, a pharmaceutical non-aqueous composition comprising a dehydrated insulinotropic peptide, andat least onesemi-polar protic organic solventwhich insulinotropic peptide has been dehydratedat a target pH which is at least 1 pH unit from the pI of the insulinotropic peptide in aqueous solution, is provided.

In a further aspect of the invention, a pharmaceutical non-aqueous composition comprising

a) a dehydrated therapeutically active insulinotropic peptide, and
b) at least onesemi-polar protic organic solvent
which insulinotropic peptide has been dehydratedat a target pH which is at least 1 pH unit from the pI of the insulinotropic peptide in aqueous solution, and said target pH is in the range from about 6.0 to about 9.0, is provided.

In another aspect of the invention, a pharmaceutical non-aqueous composition comprising a mixture of

a) a dehydrated therapeutically active insulinotropic peptide, and

b) at least onesemi-polar protic organic solvent
which insulinotropic peptide has been dehydratedat a target pH which is at least 1 pH unit from the pI of the insulinotropic peptide in aqueous solution. In a preferred embodiment, thetarget pH is in the range from about 6.0 to about 9.0.

The term "non-aqueous" as used herein refers to a composition which comprises less than 10% w/w water. In a more preferred embodiment, the composition according to the invention comprises less than8% w/w water, in a more preferred embodiment less than 5% w/w water, in a more preferred embodiment less than 3% w/w water and in an even more preferred embodiment less than 2% w/w water.

The term "dehydrated" as used herein in connection with a insulinotropic peptiderefers to a insulinotropic peptide which has been dried from an aqueous solution. The term "target pH" as used herein refers to the aqueous pH which will establish when dehydrated insulinotropic peptide is rehydrated in pure water to a concentration of approximately 40 mg/ml or more.The target pH will typically be identical to the pH of the aqueous insulinotropic peptide solution from which the insulinotropic peptide was recovered by drying. However, the pH of the insulinotropic peptide solution will not be identical to the target pH, if the insulinotropic peptide solution contains volatile acids or bases. It has been found that the pH history of the insulinotropic peptide will be determinant for the amount of the insulinotropic peptide, which can be solubilized in the semi-polar protic organic solvent.

According to the invention the insulinotropic peptide has been dehydratedat a target pH which is at least 1 pH unit from the pI of the insulinotropic peptide in aqueous solution. Thus, in one aspect of the invention, the target pH is more than 1 pH unit above the isoelectric point of the insulinotropic peptide. In another aspect of the invention, the target pH is more than 1 pH unit below the isoelectric point of the insulinotropic peptide. In a preferred aspect, the target pH is more than 1.5 pH units above or below the pI of the insulinotropic peptide. In an even more preferred aspect, the target pH is 2.0 pH units above or below the pI of the insulinotropic peptide. In a further aspect, the target pH is 2.5 pH units above or below the pI of the insulinotropic peptide. In yet a further aspect, the target pH is above the pI of the insulinotropic peptide.

By "volatile base" is meant a base, which to some extend will evaporate upon heating and/or at reduced pressure, e.g. bases which have a vapour pressure above 65 Pa at room temperature or an aqueous azeotropic mixture including a base having a vapour pressure above 65 Pa at room temperature. Examples of volatile bases are ammonium hydroxides, tetraalkylammonium hydroxides, secondary amines, tertiary amines, aryl amines, alphatic amines or ammonium bicarbonate or a combination. For example the volatile base can be bicarbonate, carbonate, ammonia, hydrazine or an organic base such as a lower aliphatic amines e.g. trimethyl amine, triethylamine, diethanolamines, triethanolamine and their salts. Further the volatile base can be ammonium hydroxide, ethyl amine or methyl amine or a combination hereof.

By "volatile acid" is meant an acid, which to some extend will evaporate upon heating and/or at reduced pressure, e.g. acids which have a vapour pressure above 65 Pa at room temperature or an aqueous azeotropic mixture including an acid having a vapour pressure above 65 Pa at room temperature. Examples of volatile acids are carbonic acid, formic acid, acetic acid, propionic acid and butyric acid.

A "non volatile base" as mentioned herein means a base, which do not evaporate or only partly evaporate upon heating, e.g. bases with a vapour pressure below 65 Pa at room temperature. The non volatile base can be selected from the group consisting of alkaline metal salts, alkaline metal hydroxides, alkaline earth metal salts, alkaline earth metal hydroxides and amino acids or a combination hereof. Examples of non-volatile bases are sodium hydroxide, potassium hydroxide, calcium hydroxide, and calcium oxide.

A "non volatile acid" as mentioned herein means an acid, which do not evaporate or only partly evaporate upon heating, e.g. bases with a vapour pressure below 65 Pa at room temperature. Examples of non-volatile acids are hydrochloric acid, phosphoric acid and sulfuric acid.

The term "the pI of the insulinotropic peptide" as used herein refers to the isoelectric point of a insulinotropic peptide.

The term "isoelectric point" as used herein means the pH value where the overall net charge of a macromolecule such as a peptide is zero. In peptides there may be several charged groups, and at the isoelectric point the sum of all these charges is zero. At a pH above the isoelectric point the overall net charge of the peptide will be negative, whereas at pH values below the isoelectric point the overall net charge of the peptide will be positive.

The pI of a protein can be determined experimentally by electrophoresis techniques such as electrofocusing:

> A pH gradient is established in an anticonvective medium, such as a polyacrylamide gel. When a protein is introduced in to the system it will migrate under influence of an electric field applied across the gel. Positive charged proteins will migrate to the cathode. Eventually, the migrating protein reaches a point in the pH gradient where its net electrical charge is zero and is said to be focused. This is the isoelectric pH (pI) of the protein. The protein is then fixed on the gel and stained. The pI of the protein can then be determined by comparison of the position of the protein on the gel relative to marker molecules with known pI values.

The net charge of a protein at a given pH value can be estimated theoretically per a person skilled in the art by conventional methods. In essence, the net charge of protein is the equivalent to the sum of the fractional charges of the charged amino acids in the protein: aspartate (β-carboxyl group), glutamate (δ-carboxyl group), cysteine (thiol group), tyrosine (phenol group), histidine (imidazole side chains), lysine (ε-ammonium group) and arginine (guanidinium group). Additonally, one should also take into account charge of protein terminal groups ($\alpha$-$NH_2$ and $\alpha$-COOH). The fractional charge of the ionisable groups can be calculated from the intrinsic pKa values.

The drying i.e. dehydrationof the insulinotropic peptidecan be performed by any conventional drying method such e.g. by spray- , freeze-, vacuum-, open - and contact drying. In one aspect of the invention, the insulinotropic peptide solution is spray dried to obtain a water content below about 10%. The water content may be below about 8%, below about 6%, below about 5%, below about 4%, below about 3%, below about 2% or below about 1% calculated on/ measured by loss on drying test (gravimetric) as stated in the experimental part.

In one aspect of the invention the insulinotropic peptide is spray dried. In a further aspect of the invention, the insulinotropic peptide is freeze-dried.

In one aspect of the invention, the solubility obtained by the pre-treatment of the insulinotropic peptide by dehydration at the selected target pH in an organic solvent is at least 20 mg/ml. In a further aspect, the solubility of dehydrated insulinotropic peptide in the organic solvent is at least 30 mg/ml. In yet a further aspect,the solubility of dehydrated insulinotropic peptide in the organic solvent is at least 40 mg/ml. In yet a further aspect, the solubility of dehydrated insulinotropic peptide in the organic solvent is at least 50 mg/ml. In yet a further aspect,the solubility of dehydrated insulinotropic peptide in the organic solvent is at least 60 mg/ml. In yet a further aspect,the solubility of dehydrated insulinotropic peptide in the organic solvent is at least 70 mg/ml. In yet a further aspect,the solubility of dehydrated insulinotropic peptide in the organic solvent is at least 80 mg/ml. In yet a further aspect,the solubility of dehydrated insulinotropic peptide in the organic solvent is at least 100 mg/ml.

The term "semi-polar protic organic solvent" as used herein refers to a hydrophilic, water miscible carbon-containing solvent that contains an O-H or N-H bond, or mixtures thereof.The polarity is reflected in the dielectric constant or the dipole moment of a solvent. The polarity of a solvent determines what type of compounds it is able to dissolve and with what other solvents or liquid compounds it is miscible. Typically, polar solvents dissolve polar compounds best and non-polar solvents dissolve non-polar compounds best: "like dissolves like". Strongly polar compounds like inorganic salts (e.g. sodium chloride) dissolve only in very polar solvents.

Semi-polar solvents are here defined as solvents with a dielectricity constant in the range of 20-50, whereas polar and non-polar solvents are defined by a dielectricity constant above 50 and below 20, respectively. Examples of semi-polar protic are listed in Table 1 together with water as a reference.

Table 1. Dielectricity constants (static permittivity) of selected semi-polar organic protic solvents and water as a reference(Handbook of Chemistry and Physics, CMC Press, dielectricity constants are measured in static electric fields or at relatively low frequencies, where no relaxation occurs)

| Solvent (Temperature , Kelvin) | Dielectricity constant, ε* |
|---|---|
| Water (293.2) | 80.1 |
| Propanetriol [Glycerol] (293.2) | 46.53 |

(continued)

| Solvent (Temperature , Kelvin) | Dielectricity constant, ε* |
|---|---|
| Ethanediol [Ethylene Glycol] (293.2) | 41.4 |
| 1,3-propanediol (293.2) | 35.1 |
| Methanol (293.2) | 33.0 |
| 1,4-butanediol (293.2) | 31.9 |
| 1,3-butanediol (293.2) | 28.8 |
| 1,2-propanediol [propylene glycol] (303.2) | 27.5 |
| Ethanol (293.2) | 25.3 |
| Isopropanol (293.2) | 20.18 |

In the present context, 1,2-propanediol and propylene glycol is used interchangeable. In the present context, propanetriol and glycerol is used interchangeably.In the present context, ethanediol and ethylene glycolis used interchangeably.

In one aspect of the invention, the solvent is selected from the group consisting of polyols. The term "polyol" as used herein refers to chemical compounds containing multiple hydroxyl groups.

In a further aspect of the invention, the solvent is selected from the group consisting of diols and triols. The term "diol" as used herein refers to chemical compounds containing twohydroxyl groups. The term "triol" as used herein refers to chemical compounds containing threehydroxyl groups.

In a further aspect of the invention, the solvent is selected from the group consisting of glycerol (propanetriol), ethanediol (ethylene glycol), 1,3-propanediol, methanol, 1,4-butanediol, 1,3-butanediol, propylene glycol (1,2-propanediol), ethanol and isopropanol, or mixtures thereof. In a further aspect of the invention, the solvent is selected from the group consisting of propylene glycol and glycerol. In a preferred aspect of the invention, the solvent is glycerol.This solvent is biocompatible at even high dosages and has a high solvent capacity for e.g. GLP-1 compounds.In another preferred aspect of the invention, the solvent is selected from the group consisting of propylene glycol and ethylene glycol. These solvents have a low viscosity, are biocompatible at moderate doses, and have very high solvent capacity for e.g. GLP-1 compounds.

The solvents should preferably be of high purity with a low content of e.g. aldehydes, ketones and other reducing impurities in order to minimize chemical deterioration of the solubilized insulinotropic peptide due to e.g. Maillard reaction. Scavenger molecules like glycylglycine and ethylenediamine may be added to the formulations comprising semi-polar protic organic solvent(s) such as polyols to reduce deterioration of the insulinotropic peptide whereas antioxidants can be added to reduce the rate of formation of further reducing impurities.

In one aspect of the invention, the organic solvent is present in the pharmaceutical composition in an amount of at least 20% w/w.In a further aspect of the invention,the organic solvent is present in an amount of at least 30% w/w. In a further aspect of the invention,the organic solvent is present in an amount of at least 40% w/w. In a further aspect of the invention,the organic solvent is present in an amount of at least 50% w/w. In a further aspect of the invention,the organic solvent is present in an amount of at least 80% w/w.

In order to increase the shelf-stability of the pharmaceutical composition it has been found that the target pH advantageously is adjusted to between about 6.0 and about 9.0. In one aspect of the invention, the target pH is between about 6.0 and about 9.0,such as between about 6.2 and about 8.4, between about 6.4 and about 8.7, between about 6.5 and about 8.5, between about 7.0 and about 8.5, or between about 7.2 and about 8.3. In one aspect the target pH is above about 7.4, above about 7.6, above about 7.8, above about 8.0, above about 8.2, above about 8.4 or above about 8.6. It is believed that the increased shelf stability is due to fewer tendencies of the insulinotropic peptides to fibrillate after having been dehydrated as described above.

The term "shelf-stable pharmaceutical composition" as used herein means a pharmaceutical composition which is stable for at least the period which is required by regulatory agencies in connection with therapeutic proteins. Preferably, a shelf-stable pharmaceutical composition is stable for at least one year at 5 °C. Shelf-stability includes chemical stability as well as physical stability.Chemical instability involves degradation of covalent bonds, such as hydrolysis, racemization, oxidation or crosslinking. Chemical stability of the formulations is evaluated by means of reverse phase (RP-HPLC) and size exclusion chromatography (SE-HPLC).In one aspect of the invention, the formation of peptide related impurities during shelf-life is less than 10 % of the total peptide content. In a further aspect of the invention, the formation of peptide related during impurities during shelf-life is less than 5 %.The RP-HPLC analysis is typically conducted in water-acetonitrile or water-ethanol mixtures. In one embodiment, the solvent in the RP-HPLC step will comprise a salt such as $Na_2SO_4$, $(NH_4)_2SO_4$, NaCl, KCl, and buffer systems such as phosphate, and citrate and maleic acid. The required concentration of salt in the solvent may be from about 0.1 M to about 1 M, preferable between 0.2 M to 0.5 M, most preferable between 0.3 to 0.4 M. Increase of the concentration of salt requires an increase in the concentration of organic solvent in order

to achieve elution from the column within a suitable time.

Physical instability involves conformational changes relative to the native structure, which includes loss of higher order structure, aggregation, fibrillation, precipitation or adsorption to surfaces. For example GLP-1 compounds are known to be prone to instability due to fibrillation.Physical stability of the formulations may be evaluated by conventional means of e.g. visual inspection, nephelometry and Thioflavin T assay after storage of the formulation at different temperatures for various time periods.

Conformational stability can be evaluated by circular dichroism and NMR as described by e.g. Hudson and Andersen, Peptide Science, vol 76 (4), pp. 298-308 (2004).

The term "therapeutically active insulinotropic peptide" or "therapeutic insulinotropic peptides" as used herein refers to aninsulinotropic peptide able to cure, alleviate or partially arrest the clinical manifestations of a given disease and its complications.

In a further aspect of the invention, the term "therapeutically active insulinotropic peptide" or "therapeutic insulinotropic peptides" as used herein means a insulinotropic peptide which is being developed for therapeutic use, or which has been developed for therapeutic use.

An amount adequate to accomplish this is defined as "therapeutically effective amount".

Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

The term "polypeptide" or "peptide" isused interchangeably herein to mean a compound composed of at least five constituent amino acids connected by peptide bonds. The constituent amino acids may be from the group of the amino acids encoded by the genetic code and they may be natural amino acids which are not encoded by the genetic code, as well as synthetic amino acids. Natural amino acids which are not encoded by the genetic code are e.g. hydroxyproline, γ-carboxyglutamate, ornithine, phosphoserine, D-alanine and D-glutamine. Synthetic amino acids comprise amino acids manufactured by chemical synthesis, i.e. D-isomers of the amino acids encoded by the genetic code such as D-alanine and D-leucine, Aib (α-aminoisobutyric acid), Abu (α-aminobutyric acid), Tle (tert-butylglycine), β-alanine, 3-aminomethyl benzoic acid, anthranilic acid.

The production of polypeptides and peptides is well known in the art. Polypeptides or peptides may for instance be produced by classical peptide synthesis, e.g. solid phase peptide synthesis using t-Boc or Fmoc chemistry or other well established techniques, see e.g. Greene and Wuts, "Protective Groups in Organic Synthesis", John Wiley & Sons, 1999. The polypeptides or peptides may also be produced by a method which comprises culturing a host cell containing a DNA sequence encoding the (poly)peptide and capable of expressing the (poly)peptide in a suitable nutrient medium under conditions permitting the expression of the peptide. For (poly)peptides comprising non-natural amino acid residues, the recombinant cell should be modified such that the non-natural amino acids are incorporated into the (poly)peptide, for instance by use of tRNA mutants.

The term "pharmaceutical composition" as used herein means a product comprising a therapeutically active insulinotropic peptidetogether with pharmaceutical excipients such as, a surfactant, buffer, preservative and tonicity modifier, said pharmaceutical composition being useful for treating, preventing or reducing the severity of a disease or disorder by administration of said pharmaceutical composition to a person. Thus a pharmaceutical composition is also known in the art as a pharmaceutical formulation. It is to be understood that pH of a pharmaceutical composition which is to be reconstituted is the pH value which is measured on the reconstituted composition produced by reconstitution in the prescribed reconstitution liquid at room temperature.

The term "pharmaceutically acceptable" as used herein means suited for normal pharmaceutical applications, i.e. giving rise to no serious adverse events in patients etc.

The term "buffer" as used herein refers to a chemical compound in a pharmaceutical composition that reduces the tendency of pH of the composition to change over time as would otherwise occur due to chemical reactions. Buffers include chemicals such as sodium phosphate, TRIS, glycine and sodium citrate.

The term "preservative" as used herein refers to a chemical compound which is added to a pharmaceutical composition to prevent or delay microbial activity (growth and metabolism). Examples of pharmaceutically acceptable preservatives are phenol, m-cresol and a mixture of phenol and m-cresol.

The term "stabilizer" as used herein refers to chemicals added to peptide containing pharmaceutical compositions in order to stabilize the peptide, i.e. to increase the shelf life and/or in-usetime of such compositions. Examples of stabilizers used in pharmaceutical formulations are L-glycine, L-histidine, arginine, glycylglycine, ethylenediamine, citrate, EDTA,polyethylene glycol, carboxymethylcellulose, and surfactants and antioxidants like alfa-tocopherol and l-ascorbic acid.

The term "surfactant" as used herein refers to any substance, in particular a detergent, that can adsorb at surfaces and interfaces, like liquid to air, liquid to liquid, liquid to container or liquid to any solid. The surfactant may be selected from a detergent, such as ethoxylated castor oil, polyglycolyzed glycerides, acetylated monoglycerides, sorbitan

fatty acid esters, polysorbate, such as polysorbate-20, poloxamers, such as poloxamer 188 and poloxamer 407, polyoxyethylene sorbitan fatty acid esters, polyoxyethylene derivatives such as alkylated and alkoxylated derivatives (tweens, e.g. Tween-20, or Tween-80), monoglycerides or ethoxylated derivatives thereof, diglycerides or polyoxyethylene derivatives thereof, glycerol, cholic acid or derivatives thereof, lecithins, alcohols and phospholipids, glycerophospholipids (lecithins, kephalins, phosphatidyl serine), glyceroglycolipids (galactopyransoide), sphingophospholipids (sphingomyelin), and sphingoglycolipids (ceramides, gangliosides), DSS (docusate sodium, CAS registry no [577-11-7]), docusate calcium, CAS registry no [128-49-4]), docusate potassium, CAS registry no [7491-09-0]), SDS (sodium dodecyl sulfate or sodium lauryl sulfate), dipalmitoyl phosphatidic acid, sodium caprylate, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, palmitoyl lysophosphatidyl-L-serine, lysophospholipids (e.g. 1-acyl-sn-glycero-3-phosphate esters of ethanolamine, choline, serine or threonine), alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the postively charged DODAC, DOTMA, DCP, BISHOP, lysophosphatidylserine and lysophosphatidylthreonine, zwitterionic surfactants (e.g. N-alkyl-N,N-dimethylammonio-1-propanesulfonates, 3-cholamido-1-propyldimethylammonio-1-propanesulfonate, dodecylphosphocholine, myristoyl lysophosphatidylcholine, hen egg lysolecithin), cationic surfactants(quarternary ammonium bases) (e.g. cetyl-trimethylammonium bromide, cetylpyridinium chloride), non-ionic surfactants (e. g. alkyl glucosides like dodecyl β-D-glucopyranoside, dodecyl β-D-maltoside, tetradecyl β-D-glucopyranoside, decyl β-D-maltoside,dodecyl α-D-maltoside, tetradecyl β-D-maltoside, hexadecyl β-D-maltoside, decyl β-D-maltotrioside,dodecyl β-D-maltotrioside, tetradecyl β-D-maltotrioside, hexadecyl β-D-maltotrioside, n-dodecyl-sucrose, n-decyl-sucrose, fatty alcohol ethoxylates (e. g. polyoxyethylene alkyl ethers like octaethylene glycol mono tridecyl ether, octaethylene glycol mono dodecyl ether,octaethylene glycol mono tetradecyl ether), block copolymers as polyethyleneoxide/polypropyleneoxide block copolymers (Pluronics/Tetronics, Triton X-100) ethoxylated sorbitan alkanoates surfactants (e. g. Tween-40, Tween-80, Brij-35), fusidic acid derivatives (e.g. sodium tauro-dihydrofusidate etc.), long-chain fatty acids and salts thereof C6-C12 (eg. oleic acid and caprylic acid), acylcarnitines and derivatives, $N^{\alpha}$-acylated derivatives of lysine, arginine or histidine, or side-chain acylated derivatives of lysine or arginine, $N^{\alpha}$-acylated derivatives of dipeptides comprising any combination of lysine, arginine or histidine and a neutral or acidic amino acid, $N^{\alpha}$-acylated derivative of a tripeptide comprising any combination of a neutral amino acid and two charged amino acids, or the surfactant may be selected from the group of imidazoline derivatives, or mixtures thereof.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder, and prevention of the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

The term "analogue" as used herein referring to a peptide means a modified peptide wherein one or more amino acid residues of the peptide have been substituted by other amino acid residues and/or wherein one or more amino acid residues have been deleted from the peptide and/or wherein one or more amino acid residues have been added to the peptide. Such addition or deletion of amino acid residues can take place at the N-terminal of the peptide and/or at the C-terminal of the peptide. In one embodiment an analogue comprises less than 6 modifications (substitutions, deletions, additions) relative to the native peptide. In another embodiment an analogue comprises less than 5 modifications (substitutions, deletions, additions) relative to the native peptide. In another embodiment an analogue comprises less than 4 modifications (substitutions, deletions, additions) relative to the native peptide. In another embodiment an analogue comprises less than 3 modifications (substitutions, deletions, additions) relative to the native peptide. In another embodiment an analogue comprises less than 2 modifications (substitutions, deletions, additions) relative to the native peptide. In another embodiment an analogue comprises only a single modification (substitutions, deletions, additions) relative to the native peptide.The added and/or exchanged amino acid residues can either be codable amino acid residues or other naturally occurring residues or purely synthetic amino acid residues.

The term "derivative" as used herein in relation to a parent peptide means a chemically modified parent protein or an analogue thereof, wherein at least one substituent is not present in the parent protein or an analogue thereof, i.e. a parent protein which has been covalently modified. Typical modifications are amides, carbohydrates, alkyl groups, acyl groups, esters, PEGylations and the like.

The terms GLP-1, GLP-2, exendin-3 and exendin-4 are known to a person skilled in the art. For example "GLP-1 compound" or "GLP-1 peptide" as used herein means GLP-1(7-37) (SEQ ID NO. 1), insulinotropic analogue thereof and insulinotropic derivatives thereof. Non-limiting examples of GLP-1 analogues are GLP-1(7-36) amide, $Arg^{34}$-GLP-1(7-37), $Gly^{8}$-GLP-1(7-37), $Val^{8}$-GLP-1(7-36)-amide and $Val^{8}Asp^{22}$-GLP-1(7-37). Non-limiting examples of GLP-1 derivatives are desamino-$His^{7}$, $Arg^{26}$, $Lys^{34}(N^{\varepsilon}$-($\gamma$-Glu($N^{\alpha}$-hexadecanoyl)))-GLP-1(7-37), desamino-$His^{7}$, $Arg^{26}$, $Lys^{34}$ ($N^{\varepsilon}$-octanoyl)-GLP-1(7-37), $Arg^{26,34}$, $Lys^{38}(N^{\varepsilon}$-($\omega$-carboxypentadecanoyl))-GLP-1(7-38), $Arg^{26,34}$, $Lys^{36}(N^{\varepsilon}$-($\gamma$-Glu ($N^{\alpha}$-hexadecanoyl)))-GLP-1(7-36) and $Arg^{34}$, $Lys^{26}(N^{\varepsilon}$-($\gamma$-Glu($N^{\alpha}$-hexadecanoyl)))-GLP-1(7-37).

The term "dipeptidyl aminopeptidase IV protected" as used herein means a compound, e.g. a GLP-1 analogue, which is more resistant to dipeptidyl aminopeptidase IV (DPP-IV) than the native compound, e.g. GLP-1(7-37). Resistance of a GLP-1 compound towards degradation by dipeptidyl aminopeptidase IV is determined by the following degradation assay:

> Aliquots of the GLP-1 compound (5 nmol) are incubated at 37 °C with 1 μL of purified dipeptidyl aminopeptidase IV corresponding to an enzymatic activity of 5 mU for 10-180 minutes in 100 μL of 0.1 M triethylamine-HCl buffer, pH 7.4. Enzymatic reactions are terminated by the addition of 5 μL of 10% trifluoroacetic acid, and the peptide degradation products are separated and quantified using HPLC analysis. One method for performing this analysis is: The mixtures are applied onto a Vydac C18 widepore (30 nm pores, 5 μm particles) 250 x 4.6 mm column and eluted at a flow rate of 1 ml/min with linear stepwise gradients of acetonitrile in 0.1% trifluoroacetic acid (0% acetonitrile for 3 min, 0-24% acetonitrile for 17 min, 24-48% acetonitrile for 1 min) according to Siegel et al., Regul. Pept. 1999;79:93-102 and Mentlein et al. Eur. J. Biochem. 1993;214:829-35. Peptides and their degradation products may be monitored by their absorbance at 220 nm (peptide bonds) or 280 nm (aromatic amino acids), and are quantified by integration of their peak areas related to those of standards. The rate of hydrolysis of a GLP-1 compound by dipeptidyl aminopeptidase IV is estimated at incubation times which result in less than 10% of the GLP-1 compound being hydrolysed.

The term "insulinotropic" as used herein referring to a peptide or a compound means the ability to stimulate secretion of insulin in response to an increased plasma glucose level. Insulinotropic peptides and compounds are agonists of the GLP-1 receptor. The insulinotropic property of a compound may be determined by in vitro or in vivo assays known in the art. The following in vitro assay may be used to determine the insulinotropic nature of a compound such as a peptide. Preferably insulinotropic compounds exhibit an $EC_{50}$ value in below assay of less than 5 nM, even more preferably EC50 values less than 500 pM.

Baby hamster kidney (BHK) cells expressing the cloned human GLP-1 receptor (BHK 467-12A) are grown in DMEM media with the addition of 100 IU/mL penicillin, 100 μL/mL streptomycin, 10% foetal calf serum and 1 mg/mL Geneticin G-418 (Life Technologies). Plasma membranes are prepared by homogenization in buffer (10 mM Tris-HCl, 30 mM NaCl and 1 mM dithiothreitol, pH 7.4, containing, in addition, 5 mg/mL leupeptin (Sigma), 5 mg/L pepstatin (Sigma), 100 mg/L bacitracin (Sigma), and 16 mg/L aprotinin (Calbiochem-Novabiochem, La Jolla, CA)). The homogenate was centrifuged on top of a layer of 41% W7v sucrose. The white band between the two layers was diluted in buffer and centrifuged. Plasma membranes were stored at -80 °C until used.

The functional receptor assay is carried out by measuring cAMP as a response to stimulation by the insulinotropic peptide or insulinotropic compound. Incubations are carried out in 96-well microtiter plates in a total volume of 140 mL and with the following final concentrations: 50 mM Tris-HCl, 1 mM EGTA, 1.5 mM $MgSO_4$, 1.7 mM ATP, 20 mM GTP, 2 mM 3-isobutyl-1-methylxanthine (IBMX), 0.01% w/v Tween-20, pH 7.4. Compounds are dissolved and diluted in buffer. GTP is freshly prepared for each experiment: 2.5 μg of membrane is added to each well and the mixture is incubated for 90 min at room temperature in the dark with shaking. The reaction is stopped by the addition of 25 mL 0.5 M HCl. Formed cAMP is measured by a scintillation proximity assay (RPA 542, Amersham, UK). A dose-response curve is plotted for the compound and the $EC_{50}$ value is calculated using GraphPad Prism software.

The term "prodrug of an insulinotropic compound" as used herein means a chemically modified compound which following administration to the patient is converted to an insulinotropic compound. Such prodrugs are typically amino acid extended versions or esters of an insulinotropic compound.

The term "exendin-4 compound" as used herein is defined as exendin-4(1-39) (SEQ ID NO. 2), insulinotropic fragments thereof, insulinotropic analogs thereof and insulinotropic derivatives thereof. Insulinotropic fragments of exendin-4 are insulinotropic peptides for which the entire sequence can be found in the sequence of exendin-4 (SEQ ID NO. 2) and where at least one terminal amino acid has been deleted. Examples of insulinotropic fragments of exendin-4(1-39) are exendin-4(1-38) and exendin-4(1-31). The insulinotropic property of a compound may be determined by in vivo or in vitro assays well known in the art. For instance, the compound may be administered to an animal and monitoring the insulin concentration over time. Insulinotropic analogs of exendin-4(1-39) refer to the respective molecules wherein one or more of the amino acids residues have been exchanged with other amino acid residues and/or from which one or more amino acid residues have been deleted and/or from which one or more amino acid residues have been added with the proviso that said analogue either is insulinotropic or is a prodrug of an insulinotropic compound. An example of

an insulinotropic analog of exendin-4(1-39) is $Ser^2Asp^3$-exendin-4(1-39) wherein the amino acid residues in position 2 and 3 have been replaced with serine and aspartic acid, respectively (this particular analog also being known in the art as exendin-3). Insulinotropic derivatives of exendin-4(1-39) and analogs thereof are what the person skilled in the art considers to be derivatives of these peptides, i.e. having at least one substituent which is not present in the parent peptide molecule with the proviso that said derivative either is insulinotropic or is a prodrug of an insulinotropic compound. Examples of substituents are amides, carbohydrates, alkyl groups, esters and lipophilic substituents. An example of an insulinotropic derivatives of exendin-4(1-39) and analogs thereof is $Tyr^{31}$-exendin-4(1-31)-amide.

The term "stable exendin-4 compound" as used herein means a chemically modified exendin-4(1-39), i.e. an analogue or a derivative which exhibits an in vivo plasma elimination half-life of at least 10 hours in man, as determined by conventional methods.

The term "dipeptidyl aminopeptidase IV protected exendin-4 compound" as used herein means an exendin-4 compound which is more resistant towards the plasma peptidase dipeptidyl aminopeptidase IV (DPP-IV) than exendin-4 (SEQ ID NO. 2), as determined by the assay described under the definition of dipeptidyl aminopeptidase IV protected GLP-1 compound.

The GLP-1 analogues may be such wherein the naturally occurring Lys at position 34 of GLP-1(7-37) has been substituted with Arg. All amino acids for which the optical isomer is not stated is to be understood to mean the L-isomer.

In aspects of the invention an analogue of the insulinotropic peptide is obtained wherein a maximum of 17 amino acids have been modified relative to the unmodified insulinotropic peptide. In aspects of the invention a maximum of 15 amino acids have been modified. In aspects of the invention a maximum of 10 amino acids have been modified. In aspects of the invention a maximum of 8 amino acids have been modified. In aspects of the invention a maximum of 7 amino acids have been modified. In aspects of the invention a maximum of 6 amino acids have been modified. In aspects of the invention a maximum of 5 amino acids have been modified. In aspects of the invention a maximum of 4 amino acids have been modified. In aspects of the invention a maximum of 3 amino acids have been modified. In aspects of the invention a maximum of 2 amino acids have been modified. In aspects of the invention 1 amino acid has been modified.

Also, derivatives of precursors or intermediates of insulinotropic peptides are covered by the invention.

In anaspect of the invention, the therapeutically active insulinotropic peptideis selected from the group consisting of insulinotropic peptide, GLP-1(7-37) or an analog or derivative thereof, exendin or an analog or derivative thereof andGLP-2 or an analog or derivative thereof.

In one aspect of the invention the insulintropic polypeptide is a glucagon-like peptide. In a further aspect the insulintropic polypeptide is selected from the group consisting of GLP-1, GLP-2, exendin-4, exendin-3 and analogues and derivatives thereof. In one aspect the glucagon-like peptide is an insulinotropic agent.

Conformational stability protein based drugs is important for maintaining biological activity and for minimizing irreversible loss of structure due to denaturation and fibrillation. Especially large insulinotropic peptides and proteins are labile with respect to conformational change due to complicated refolding patterns. Also, insulinotropic peptides with a known history of fibrillation, such as GLP-1, are particularly sensitive towards destabilization of tertiary structure (i.e. formation of a molten globular state).

Effective amounts for each purpose will depend on the severity of the disease or injury as well as the weight and general state of the subject.

In one aspect of the invention, the pharmaceutical formulation comprises a therapeutically active insulinotropic peptide in a concentration from 0.1 % w/w to 50 % w/w.

Effective amounts for each purpose will depend on the severity of the disease or injury as well as the weight and general state of the subject.

The term "about" as used herein means in reasonable vicinity of the stated numerical value, such as plus or minus 10%.

The present invention further provides a process for the preparation of a pharmaceutical solution by:

a) providing an aqueous solution of a therapeutically active insulinotropic peptide optionally comprising excipients,

b) adjusting the pH value to a target pH value which is 1 unit, preferably 2 units and more preferably 2.5 pH units above or below the pI of the insulinotropic peptide,

c) removing water (dehydrating) the insulinotropic peptide by conventional drying technologies such as freeze- and spray drying, and

d) mixing and dissolution of the insulinotropic peptide in a semi-polar protic non-aqueous solvent e.g. by stirring, tumbling or other mixing methods,

e) optionally filtration or centrifugation of the non-aqueous insulinotropic peptide solution to remove non-dissolved inorganic salts,

f) optionally removing residual amounts of waters by e.g. adding solid dessicants or vacuum drying,

g) optionally adding further excipients such as a hydrofluoroalkane propellants and cosolvents for solution pressurized metered dose inhalers or adding detergents, polymers, lipids and co-solvents for oral dosage forms.

The present invention further provides a process for the preparation of a pharmaceutical composition by:

a) providing an aqueous solution of a therapeutically active insulinotropic peptide, optionally containing stabilizers such as zinc and glycylglycine,

b) adjusting the pH value to 1 unit, preferably 2 units and more preferably 2.5 pH units above or below the pI of the insulinotropic peptidee.g. by adding a non-volatile base or a acid, such as hydrochloric acid or sodium hydroxide, to the solution

c) removing water (dehydrating) the insulinotropic peptide by conventional drying technologies such as freeze- and spray drying,

d) mixing and dissolution of the insulinotropic peptide in a semi-polar protic non-aqueous solvent e.g. by stirring, tumbling or other mixing methods,

e) optionally filtration or centrifugation of the non-aqueous insulinotropic peptide solution to remove non-dissolved inorganic salts,

f) optionally removing residual amounts of waters by e.g. adding solid dessicants or vacuum drying,

g) optionally adding further excipients such as a hydrofluoroalkane propellants and cosolvents for solution pressurized metered dose inhalers or adding detergents, polymers, lipids and co-solvents for oral dosage forms.

In one aspect of the invention, the insulinotropic peptide is added to an aqueous solution. The aqueous solution can be pure water or it can contain excipients or an alkaline solution.

In one aspect of the invention, the pH of the insulinotropic peptide solution is adjusted with an alkaline solution comprising a non-volatile base. The non volatile base can be selected from the group consisting of alkaline metal salts, alkaline metal hydroxides, alkaline earth metal salts, alkaline earth metal hydroxides and amino acids or a combination hereof. For example the pH can be adjusted with sodium hydroxide, potassium hydroxide, calcium hydroxide, calcium oxide or any combination hereof. In another aspect of the invention, the pH of the insulinotropic peptide solution is adjusted with anon-volatile acid selected from hydrochloric acid, phosphoric acid and sulfuric acid.

In one aspect of the invention, the insulinotropic peptide non-aqueous solution comprises glycylglycine. Glycylglycine might act as a scavenger for reductive impurities in the solvent such as e.g. glyceraldehyde. In one aspect glycylglycine is added to a propylene glycol solution comprising insulin e.g. in a concentration of glycylglycine in the solution ofbetween about 4 mM and about 200 mM.

In one aspect of the invention, the insulinotropic peptidenon-aqueous solution comprises Tween 80 and oleic acid. When Tween 80 and oleic acid is added to a non-aqueousinsulinotropic peptidesolution, the insulinotropic peptide solution forms a microemulsion upon dilution with an aqueous liquid following oral administration. The microemulsion might act as an absorption enhancer and furthermore generate more reproducible absorption kinetics. The concentration of Tween 80 in the solution will e.g. be between about 30 and 70 % w/w and the concentration of oleic acid will e.g. be between 10 and 30 % w/w.

In one aspect, the invention is related to a pharmaceutical composition for the treatment of type 1 diabetes, type 2 diabetes and other states that cause hyperglycaemia in patients in need of such a treatment.

In one aspect, the invention is related to a pharmaceutical composition according to the invention with a pharmaceutically acceptable carrier and/or a pharmaceutically acceptable additive, which composition can be provided for the treatment of type 1 diabetes, type 2 diabetes and other states that cause hyperglycaemia in patients in need of such a treatment.

In one aspect of the invention, there is provided a method of treating type 1 diabetes, type 2 diabetes and other states that cause hyperglycaemia in a patient in need of such a treatment, comprising administering to the patient a therapeutically effective amount of a pharmaceutical composition according to the invention.

In one aspect of the invention, there is provided a method of treating type 1 diabetes, type 2 diabetes and other states that cause hyperglycaemia in a patient in need of such a treatment, comprising administering to the patient a therapeutically effective amount of an pharmaceutical composition according to the invention, optionally together with a pharmaceutically acceptable carrier and/or pharmaceutical acceptable additives.

In one aspect of the invention, there is provided a method for the manufacture of a pharmaceutical composition according to the invention for the use in the treatment of type 1 diabetes, type 2 diabetes and other states that cause hyperglycaemia.

In one aspect of the invention, there is provided a method for the manufacture of a pharmaceutical composition for the use in the treatment of type 1 diabetes, type 2 diabetes and other states that cause hyperglycaemia.

## PHARMACEUTICAL COMPOSITIONS

The compositions according to the invention can, for example, be administered subcutaneously, orally, nasally or pulmonary.

For subcutaneous administration, the composition according to the invention is formulated analogously with the formulation of known therapeutically active insulinotropic peptides. Furthermore, for subcutaneous administration, the compositions according to the invention are administered analogously with the administration of known therapeutically active insulinotropic peptides and, generally, the physicians are familiar with this procedure.

In one aspect of the invention, the compositions according to the invention may be administered by inhalation in a dose effective manner to increase circulating insulin peptidelevels and/or to lower circulating glucose levels. Such administration can be effective for treating disorders such as diabetes or hyperglycaemia. Achieving effective doses of e.g. insulinotropic peptides requires administration of an inhaled dose of a composition according to the invention comprising more than about 0.5 μg/kg to about 50 μg/kginsulinotropic peptide. A therapeutically effective amount can be determined by a knowledgeable practitioner, who will take into account factors including insulin level, blood glucose levels, the physical condition of the patient, the patient's pulmonary status, or the like.

A composition according the invention may be delivered by inhalation to achieve rapid absorption of the therapeutically active insulinotropic peptide. Administration by inhalation can result in pharmacokinetics comparable to subcutaneous administration of insulinotropic peptides. Different inhalation devices typically provide similar pharmacokinetics when similar particle sizes and similar levels of lung deposition are compared.

According to the invention, a composition may be delivered by any of a variety of inhalation devices known in the art for administration of a therapeutic agent by inhalation. These devices include metered dose inhalers, nebulizers, dry powder generators, sprayers, and the like. A composition of this invention is in one aspect delivered by a pressurized metered dose inhaler or a sprayer. There are a several desirable features of an inhalation device for administering a composition according to the invention. For example, delivery by the inhalation device is advantageously reliable, reproducible, and accurate. The inhalation device should deliver small particles, for example particles less than about 10 μm, for example about 1-5 μm, for good respirability. Some specific examples of commercially available inhalation devices suitable for the practice of this invention are Turbohaler™ (Astra), Rotahaler® (Glaxo), Diskus® (Glaxo), Spiros™ inhaler (Dura), devices marketed by Nektar, 3M Drug Delivery Systems andBespak, AERx™ (Aradigm), the Ultravent® nebulizer (Mallinckrodt), the Acorn II® nebulizer (Marquest Medical Products), the Ventolin® metered dose inhaler (Glaxo), the Spinhaler® powder inhaler (Fisons), or the like.

Pressurised metered dose inhalers (pMDIs) are well known devices for administering pharmaceutical products to the respiratory tract by inhalation. pMDIs comprise a pressure resistant aerosol canister typically filled with a product such as a drug dissolved in a liquefied propellant (solution formulations) or micronized particles suspended in a liquefied propellant (suspension formulations) where the container is fitted with a metering valve. Solution formulations offer the advantage of being homogeneous with the active ingredient and excipients completely dissolved in the propellant vehicle or its mixture with suitable co-solvents such as ethanol. Solution formulations also obviate physical stability problems associated with suspension formulations so assuring more consistent uniform dosage administration. Hydrofluoroalkanes and in particular 1,1,1,2-tetrafluoroethane (HFA 134a) and 1,1,1,2,3,3,3-heptafluoropropane (HFA 227) and their mixtures havebeen acknowledged to be the best candidates for non-CFC propellants. Actuation of the metering valve allows a small portion of the spray product to be released whereby the pressure of the liquefied propellant carries the dissolved or micronized drug particles out of the container to the patient. The valve actuator is used to direct the aerosol spray into the patient's oropharynx. The canisters may consist of aluminium or stainless steel having the internal surface coated with an inert organic coating, fitted with a metering valve having a 63 microlitermetering chamber provided with a butyl rubber gasket as described in WO 03/078538.

As those skilled in the art will recognize, the formulation of the invention, the quantity of the formulation delivered, and the duration of administration of a single dose depend on the type of inhalation device employed. For some aerosol delivery systems, such as nebulizers, the frequency of administration and length of time for which the system is activated will depend mainly on the concentration of therapeutic insulinotropic peptidein the aerosol.

For example, shorter periods of administration can be used at higher concentrations of therapeutic insulinotropic peptide in the nebulizer solution. Devices such as metered dose inhalers can produce higher aerosol concentrations, and can be operated for shorter periods to deliver the desired amount of insulinotropic peptide. Devices such as powder inhalers deliver active agent until a given charge of agent is expelled from the device. In this type of inhaler, the amount of composition according to the invention in a given quantity of the powder determines the dose delivered in a single administration.

The particle size of aninsulinotropic peptidein the formulation delivered by the inhalation device is critical with respect to the ability of the peptide to make it into the lungs, and into the lower airways or alveoli. The insulinotropic peptide in the composition of this invention can be formulated so that at least about 10% of the insulinotropic peptide delivered is deposited in the lung, for example about 10 to about 20%, or more. It is known that the maximum efficiency of pulmonary deposition for mouth breathing humans is obtained with particle sizes of about 2 μm to about 3 μm. Pulmonary deposition decreases substantially when particle sizes are above about 5 μm. Particle sizes below about 1 μm cause pulmonary deposition to decrease, and it becomes difficult to deliver particles with sufficient mass to be therapeutically effective. Thus, particles of insulinotropic peptide delivered by inhalation have a particle size less than about 10 μm, for example in the range of about 1 μm to about 5 μm. The formulation of insulinotropic peptide is selected to yield the desired particle size in the chosen inhalation device.

The insulinotropic peptidesolution may optionally be combined with pharmaceutical carriers or excipients which are suitable for respiratory and pulmonary administration. Such carriers may serve simply as bulking agents when it is desired to reduce the therapeutic insulinotropic peptide such as insulin concentration in the liquid which is being delivered to a patient, but may also serve to enhance the stability of the compositions.

Suitable materials include carbohydrates, e.g., (a) monosaccharides such as fructose, galactose, glucose, D-mannose, sorbose, and the like; disaccharides, such as lactose, trehalose, cellobiose, and the like; cyclodextrins, such as 2-hydroxypropylcyclodextrin; and polysaccharides, such as raffinose, maltodextrins, dextrans, and the like; (b) amino acids, such as glycine, arginine, aspartic acid, glutamic acid, cysteine, lysine, histidine, arginine and the like; (c) organic salts prepared from organic acids and bases, such as sodium citrate, sodium ascorbate, magnesium gluconate, sodium gluconate, tromethamine hydrochloride, and the like; (d) peptides and proteins, such as aspartame, human serum albumin, gelatin, and the like; (e) alditols, such as mannitol, xylitol, and the like. A preferred group of carriers includes lactose, trehalose, raffinose, maltodextrins, glycine, sodium citrate, tromethamine hydrochloride, human serum albumin, and mannitol.

Such carrier materials may be combined with the therapeutic insulinotropic peptide such as insulin peptide prior to dehydration such as spray drying, i.e., by adding the carrier material to the insulinotropic peptidesolution which is prepared for spray drying. In that way, the carrier material will be formed simultaneously with and as part of the protein particles.

Typically, when the carrier is formed by spray drying together with the insulinotropic peptide, the insulinotropic peptide will be present in each individual particle at a weight percent in the range from 5% to 95%, preferably from 20% to 80%. The remainder of the particle will primarily be carrier material (typically being from 5% to 95%, usually being from 20% to 80% by weight), but will also include buffer (s) may include other components as described above.

Non-aqueousinsulinotropic peptide formulations may also be combined with other active components. For example, it may be desirable to combine small amounts of amylin or active amylin analogues in the insulin formulations to improve the treatment of diabetes. Amylin is a hormone which is secreted with insulin from the pancreatic 0-cells in normal (non-diabetic) individuals. It is believed that amylin modulates insulin activity in vivo, and it has been proposed that simultaneous administration of amylin with insulin could improve blood glucose control. Combining amylin with insulin in the compositions of the present invention will provide a particularly convenient product for achieving such simultaneous administration. Amylin may be combined with insulin at from 0.1% by weight to 10% by weight (based on the total weight of insulin in a dose), preferably from 0.5% by weight to 2.5% by weight. Amylin is available from commercial suppliers, such as Amylin Corporation, San Diego, California, and can be readily formulated in the compositions of the present invention. For example, amylin may be dissolved in aqueous or other suitable solutions together with the insulin, and optionally carriers, and the solution spray dried to produce the powder product.

A spray including the spray-dried insulinotropic peptide can be produced by forcing a suspension or solution of insulinotropic peptide through a nozzle under pressure. The nozzle size and configuration, the applied pressure, and the liquid feed rate can be chosen to achieve the desired output and particle size. An electrospray can be produced, for example, by an electric field in connection with a capillary or nozzle feed.

Advantageously, particles of e.g. insulin delivered by a sprayer have a particle size less than about 10 μm, for example in the range of about 1 μm to about 5 μm.

Pharmaceutical compositions according to the present invention containing a dehydrated such as spray-dried insulinotropic peptidedissolved in a semi-polar protic organic solventsuch as in a polyolmay also be administered parenterally to patients in need of such a treatment.

Parenteral administration may be performed by subcutaneous, intramuscular or intravenous injection by means

of a syringe, optionally a pen-like syringe. Alternatively, parenteral administration can be performed by means of an infusion pump.

The preparations of the invention may be used in connection with pumps such as insulin pumps. The insulin pumpsmay be prefixed and disposable, or the insulin preparations may be supplied froma reservoir which is removable. Insulin pumps may be skinmounted, implanted or carried, and the path of the insulin preparation from the storage compartment of the pump to the patient may be more or less tortuous. Non-limiting examples of insulin pumps are disclosed in US 5,957,895, US 5, 858, 001, US4., 468, 221, US 4,468, 221, US 5, 957, 895, US 5,858, 001, US6,074,US US5, 858, 001, US 5,527,288, and US 6, 074, 369.

Injectable compositionsaccording to the present invention of the dehydrated such as spray-dried insulinotropic peptide can be prepared using the conventional techniques of the pharmaceutical industry which involve dissolving and mixing the ingredients in a semi-polar protic organic solventsuch as a polyol as appropriate to give the desired end product.

Currently, injection is the typical mode of administering a biologically active protein to the systemic circulation. The recipient may experience discomfort or pain by injection. For this reason and others there may be problems with patient compliance using injection as a mode of administration. One alternative to injection is the peroral administration of biologically active insulinotropic peptides. In the case of insulin, oral delivery may have advantages beyond convenience and compliance issues. Insulin absorbed in the gastrointestinal tract mimics the physiology of insulin secreted by the pancreas because both are released into the portal vein and carried directly to the liver. Absorption into the portal circulation maintains a peripheral-portal insulin gradient that regulates insulin secretion. In its first passage through the liver, roughly 60% of the insulin is retained and metabolized, thereby reducing the incidence of peripheral hyperinsulinemia, a factor in diabetes related systemic complications. A feared and not uncommon complication of insulin treatment and other oral antidiabetic agents is hypoglycemia.

However, peroral bioavailability of insulinotropic peptides is extremely low due to extensive proteolytic degradation in the gastrointestinal tract and low epithelial permeability.. Hence, stabilization of insulinotropic peptides against proteolytic degradation by protein engineering, such as pegylation and mutation of hot spots, is a commonly used strategy to improve oral bioavailability. Another strategy to reduce proteolytic degradation is to incorporate the insulinotropic peptide into nanoparticles, microparticles, microemulsions, microemulsion pre-concentrates, liposomes and the like. The drug is not only protected from the hostile environment in the stomach and gastrointestinal tract but also these particles might be taken up from the enteral route into the systemic circulation via the Peyers patches.

In one embodiment, the pharmaceutical non-aqueous compositionis a microemulsion pre-concentrate. This is a composition which spontaneously self emulsifies upon dilution with an aqueous medium, e.g. water or gastrointestinal juice into for example an oil-in-water (o/w) microemulsion. Microemulsions are being understood as being thermodynamically stable systems with a mean domain size of the emulsified phase in the lower nm range, for example between 20-400nm.

In general, the microemulsion pre-concentrate comprises one or more hydrophilic components, one or more lipophilic components, one or more non-ionic surfactants and/or co-surfactants and an active ingredient e.g. a therapeutic peptide or protein completely dissolved in the pre-concentrate mixture. In addition, the microemulsion pre-concentrate may, if appropriate, comprise conventional adjuvants and additives. These self (micro)-emulsifying pre-concentrates can be ingested with the expectation that a microemulsion forms in the gastrointestinal tract. The microemulsion pre-concentrate may be administered encapsulated in an enteric coated soft gelatine capsule.

Many microemulsions have been reported to enhance the bioavailability of several therapeutic compounds after oral administration.

An example of a composition of a microemulsion pre-concentrate is:

a) at least one semi-polar protic organic solvent such as a polyol (e.g. propylene glycol and/or glycerol)
b) one or more hydrophilic components such as glycerol and/or propylene glycol
b) one or more lipophilic components such as oils (e.g. mono-diglyceridesand/or triglycerides)
c) one or more non-ionic surfactants such as poloxamers and/or polysorbates
d) one or more co-surfactants such as ethanol
e) a dehydrated therapeutically active insulinotropic peptide(e.g. GLP1)
f) one or more additives such as antioxidants and/or stabilizers.

Pharmaceutical compositions according to the present invention containing a dehydrated such as a spray-dried insulinotropic peptidedissolved in a semi-polar protic organic solvent such as in a polyol may also be administered bucally or sublingually in the form ofaerosolised spray to patients in need of such a treatment.The Oral-lyn™ formulation developed by Generex Biotechnology Corporation is an example of abuccal aerosol formulation administered via a pressurized metered dose inhaler (pMDI).

Hydrofluoroalkanes (HFA), such as HFA 134a and 227, are typically used as propellants. Insulinotropic peptides are typically highly soluble in water at pH values more than 1 unit below or above pI. Since water is extremely poorly

miscible with HFA propellantsit is advantageous to use a solvent such as propylene glycol and ethanol for the protein. Said solvent is miscible with HFA propellants. Solution-type metered dose inhalers are generally preferred relative to suspension-type pMDI, which tend to have a higher variation in dose uniformity due to creaming or sedimentation of particles.

The solution type pMDI may be formulated with stabilisers and other additives, such as permeation enhancers in addition to the insulinotropic peptide.

Instead of a pressurized system, the insulinotropic peptide may alternatively be administered buccally or sublinguallyas a pumpable, such as the Coro Nitro Pump Spray® for sublingual administration of nitroglycerin.

In order to avoid lung deposition the oral spray aerosol should preferably have droplet diameters larger than 10 μm.

Mucoadhesive drug delivery systems have gained large interest in non-invasive protein/peptide delivery. They may increase the residence time of the delivery system on mucosal tissues. Several bioadhesive delivery systems have been reported to enhance the bioavailability of proteins and peptides for example after oral administration.

In a preferred embodiment, a non-aqueous freebioadhesive compositioncomprises a active protein or peptide, at least one bioadhesive polymer, and one or more semi-polar protic organic solventsuch as a polyol(s). In addition, the composition may, if appropriate, comprise conventional adjuvants and additives. The bioadhesive polymer may for example be poly(ethylene oxide)-poly(propylene oxide)-poly(ethylene oxide) block copolymers (such as Poloxamers), polyvinylpyrrolidone (such as Povidone or PVP), polyacrylic acid, polycarbophil (such as Carbopol or Carbomer), chitosan, all kinds of thiolated polymers (such as Thiomers), cellulose, alginates, hydroxyethylcellulose or other bioadhesive polymers.

The composition can be a liquid, semi-solid or a solid dosage form.

These novel compositions would have the advantage compared to for example conventional bioadhesive hydrogels, that the required aqueous medium in these formulations can be avoided and thereof increased storage stability as well as increased bioavailabilty (triggered by the polyol) will be achieved.

An example of a bioadhesive composition:

a) a dehydrated therapeutically active insulinotropic peptide(e.g. GLP1)
b) at least one semi-polar protic organic solvent such a polyol (e.g. propylene glycol and/or glycerol)
c) bioadhesive polymer (e.g. Povidone and/or Poloxamer)
d) Additives

In one embodiment, a non-aqueous composition contains a combination of a dehydrated therapeutically active insulinotropic peptide, at least one semi-polar protic organic solventsuch as polyol and one or more conventional permeation enhancers. Examples of permeation enhancers are but not limited to fatty acids, palmitoyl carnitine chloride, ethylene glycol-bis(beta-aminoethyl ether)-N,N,N',N'-tetraacetic acid (EGTA), EDTA, bile salts, ionic- as well as non-ionic surfactants such as phospholipids and alkylglycosides, polymers, chitosan, citric acid and sodium salicylate. In addition, the composition may, if appropriate, comprise conventional adjuvants and additives.

The compositions of the present invention can be administered in a format selected from e.g. a tablet, a capsule, a suppository, a liquid, a spray or an aerosol for buccal and peroral administration.

In a preferred embodiment, solid dosage forms based on semi-polar protic organic solvent(s) such aspolyols comprises at least one polyol and one or more at room temperature solid excipients for example a poloxamer, PEG or PEG stearate.

In another embodiment, the composition contains a dehydrated therapeutically active insulinotropic peptide, at least one semi-polar protic organic solventsuch as at least one polyol, and at least one at room temperature solid non ionic excipient for example poloxamers or polyoxyethylene glycols or a mixture of solid excipients. Examples of solid poloxamers are Pluronic F-127, Pluronic F-68. Examples of solid PEGs are PEG 3350, PEG 4000, PEG 8000. In addition, the solid composition may, if appropriate, comprise conventional adjuvants and additives such as binders, glidants and lubricants to ensure efficient tabletting; and disintegrants to ensure that the tablet breaks up in the digestive tract.

Tablets and capsules may be coated with a entero coating, which ruptures or becomes permeable upon contacting an aqueous environment of a defined pH, make it possible for contents of the dosage format to be selectively released at a desired site in the gastro-intestinal tract (e.g. small and large intestine) by selecting the a suitable pH-soluble polymer for a specific region. Examples of suitable enteric polymers include but are not limited to cellulose acetate phthalate, hydroxypropylmethylcellulose phthalate, polyvinylacetate phthalate, hydroxypropylmethylcellulose phthalate, methacrylic acid copolymer, shellac, methylcellulose phthalate, cellulose acetate trimellitate, hydroxypropylmethylcellulose acetate succinate, cellulose acetate phthalate, cellulose acetate succinate, cellulose acetate malate, cellulose benzoate phthalate, cellulose propionate phthalate, carboxymethylethylcellulose, ethylhydroxyethylcellulose phthalate, shellac, styrene- acrylic acid copolymer, methyl acrylate-acrylic acid copolymer, methyl acrylate- methacrylic acid copolymer, butyl acrylate-styrene-acrylic acid copolymer, methacrylic acid-methyl methacrylate copolymer, methacrylic acid-ethyl acrylate copolymer, methyl acrylate-methacrylic acid-octyl acrylate copolymer, vinyl acetate-maleic acid anhydride copolymer, styrene-maleic acid anhydride copolymer, styrene-maleic acid monoester copolymer, vinyl methyl ether-maleic acid anhydride copolymer, ethylene-maleic acid anhydride copolymer, vinyl butyl ether-maleic acid anhydride copolymer, acrylonitrile- methyl acrylate-maleic acid anhydride copolymer, butyl acrylate-styrene-maleic acid anhydride copolymer, polyvinyl alcohol phthalate, polyvinyl acetal phthalate, polyvinyl butylate phthalate and polyvinyl acetoacetal phthalate, or combinations thereof.

In a preferred embodiment the dosage form is soft gelatine capsule containing a liquid.

The invention also relates to the following embodiments:

1. Pharmaceutical non-aqueous composition comprising a mixture of

   a) a dehydrated therapeutically active insulinotropic peptide, and
   b) at least onesemi-polar protic organic solvent
   which insulinotropic peptide has been dehydratedat a target pH which is at least 1 pH unit from the pI of the insulinotropic peptide in aqueous solution, and where said target pH preferably is in the range from about 6.0 to about 9.0.

2. The pharmaceutical composition according to embodiment 1, wherein the organic solvent is selected from the group consisting of polyols.

3. The pharmaceutical composition according to embodiment 2, wherein the organic solvent is selected from the group consisting of diols and triols.

4. The pharmaceutical composition according to embodiment 3, wherein the organic solvent is selected from the group consisting of propylene glycol and glycerol.

5. The pharmaceutical composition according to embodiment 4, wherein the organic solvent is propylene glycol.

6. The pharmaceutical composition according to embodiment 4, wherein the organic solvent is glycerol.

7. The pharmaceutical composition according to any one of embodiments 1-6, wherein the insulinotropic peptide is spray dried.

8. The pharmaceutical composition according to any one of embodiments 1-6, wherein the insulinotropic peptide is freeze-dried.

9. The pharmaceutical composition according to any one of embodiments 1-8, wherein the insulinotropic peptide has been dehydrated at a pH which is at least 1.5 pH unit from the pI of the insulinotropic peptide.

10. The pharmaceutical composition according to any one of embodiments 1-9, wherein the insulinotropic peptide has been dehydrated at a pH which is at least 2 pH unit from the pI of the insulinotropic peptide.

11. The pharmaceutical composition according to any one of embodiments 1-10, wherein the insulinotropic peptide has been dehydrated at a pH which is at least 2.5 pH unit above the pI of the insulinotropic peptide.

12. The pharmaceutical composition according to any one of embodiments 1-11, wherein the solubility of dehydrated insulinotropic peptide in the organic solvent is at least 20 mg/ml.

13. The pharmaceutical composition according to any one of embodiments 1-11, wherein the solubility of dehydrated insulinotropic peptide in the organic solvent is at least 30 mg/ml.

14. The pharmaceutical composition according to any one of embodiments 1-11, wherein the solubility of dehydrated insulinotropic peptide in the organic solvent is at least 40 mg/ml.

15. The pharmaceutical composition according to any one of embodiments 1-11, wherein the solubility of dehydrated insulinotropic peptide in the organic solvent is at least 50 mg/ml.

16. The pharmaceutical composition according to any one of embodiments 1-11, wherein the solubility of dehydrated

insulinotropic peptide in the organic solvent is at least 60 mg/ml.

17. The pharmaceutical composition according to any one of embodiments 1-16, wherein thetarget pH is in the range from about 8.5 to about 11.0.

18. The pharmaceutical composition according to any one of embodiments 1-16, wherein the target pH is in the range from about 9.0 to about 10.5.

19. The pharmaceutical composition according to any one of embodiments 1-16, wherein the target pH is in the range from about 7.0 to about 8.5.

20. The pharmaceutical composition according to any one of embodiments 1-19, wherein the organic solvent is present in an amount of at least 20% w/w.

21. The pharmaceutical composition according to any one of embodiments 1-19, wherein the organic solvent is present in an amount of at least 30% w/w.

22. The pharmaceutical composition according to any one of embodiments 1-19, wherein the organic solvent is present in an amount of at least 40% w/w.

23. The pharmaceutical composition according to any one of embodiments 1-19, wherein the organic solvent is present in an amount of at least 50% w/w.

24. The pharmaceutical composition according to any one of embodiments 1-19, wherein the organic solvent is present in an amount of at least 80% w/w.

25. The pharmaceutical composition according to any one of embodiments 1-24, which comprises less than 10% w/w water.

26. The pharmaceutical composition according to any one of embodiments 1-24, which comprises less than 5% w/w water.

27. The pharmaceutical composition according to any one of embodiments 1-24, which comprises less than 2% w/w water.

28. The pharmaceutical composition according to any one of embodiments 1-27, wherein the composition is for pulmonary, parenteral, nasal or oral treatment of diabetes or hyperglycaemia.

29. The pharmaceutical composition according to any one of embodiments 1-27, wherein the composition is for pulmonary treatment of diabetes or hyperglycaemia.

30. The pharmaceutical composition according to any one of embodiments 1-27, wherein the composition is for oral treatment of diabetes or hyperglycaemia.

31. The pharmaceutical composition according to any one of embodiments 1-30, wherein the insulinotropic peptide is selected from the group consisting of GLP-1, GLP-2, Exendin-3, Exendin-4 and analogues and derivatives thereof.

32. The pharmaceutical composition according to any one of the precedingembodiments, wherein the insulinotropic peptide is a DPP-IV protected peptide.

33. The pharmaceutical composition according to any one of the precedingembodiments, wherein said insulinotropic peptide comprises a lipophilic substituent selected from the group consisting of $CH_3(CH_2)_nCO$- wherein n is 4 to 38, and $HOOC(CH_2)_mCO$- wherein m is from 4 to 38.

34. The pharmaceutical composition according to any one of the preceding embodiments, wherein said insulinotropic peptide is acylated GLP-1 or an acylated GLP-1 analogue.

35. The pharmaceutical composition according to embodiment 34, wherein said GLP-1 analogue is selected from

the group consisting of:GLP-1(7-37); Arg34-GLP-1(7-37); Aib8,Arg34-GLP-1(7-37); Aib8,Aib22,Arg34-GLP-1(7-37); Arg34-GLP-1(7-37); [3-(4-Imidazolyl)propionyl7,Arg34]GLP-1-(7-37); Gly8,Arg34-GLP-1(7-37); Aib8,Arg34,Pro37-GLP-1(7-37); Aib8,Aib22,Aib27,Aib30,Arg34,Aib35,Pro37- GLP-1 (7-37)amide; Arg26,Lys38-GLP-1(7-38); Arg26, Arg34,Lys38-GLP-1(7-38); Arg26,Arg34,Lys36,Lys38-GLP-1(7-38); Gly8,Arg26,Lys38-GLP-1(7-38); Gly8,Arg26, Arg34,Lys36,Lys38-GLP-1(7-38); DesaminoHis7Glu22Arg26Arg34Lys37-GLP-1(7-37); desaminoHis7,Glu22, Arg26,Arg34,Lys37-GLP-1(7-37)amide; Aib8,Glu22,Arg26,Arg34,Lys37-GLP-1-(7-37)amide; desaminoHis7, Glu22,Arg26,Arg 34,Phe(m-CF3)28-GLP-1-(7-37)amide; Aib8,Glu22,Arg26,Lys30-GLP-1-(7-37); Aib8,Glu22, Arg26,Lys31-GLP-1-(7-37); Aib8,Glu22,Arg26,Lys31,Arg34-GLP-1-(7-37); Aib8,Glu22,Arg26,Arg34,Lys37-GLP-1-(7-37)amide; desaminoHis7,Glu22,Arg26,Arg34,Lys37-GLP-1-(7-37)amide; DesaminoHis7,Glu22,Arg26,Glu30, Arg34,Lys37]GLP-1-(7-37); Aib8,Lys20,Glu22,Arg26,Glu30,Pro37]GLP-1-(7-37)amide; Aib8,Glu22,Arg26,Arg34, Lys37]GLP-1-(7-37)amide; DesaminoHis7,Glu22,Arg26,Arg34,Lys37]GLP-1-(7-37)amide; Aib8,Glu22,Arg26, Glu30,Pro37, Lys 38-GLP-1-(7-38); DesaminoHis7,Glu22,Arg26,Arg34,Lys37]GLP-1 (7-37) amide; DesaminoHis7, Glu22,Arg26,Arg34,Lys37]GLP-1(7-37)-amide; desaminoHis7,Glu22,Arg26, Glu30,Arg34,Lys37] (GLP-1-(7-37) amide; desaminoHis7,Glu22, Arg26,Arg34,Lys 37] (GLP-1-(7-37)amide; desaminoHis7,Glu22,Arg26,Arg34,Lys37] GLP-1 (7-37)amide; Aib8,Glu22,Arg26,Glu30,Lys36] GLP-1-(7-37)Glu-amide; DesaminoHis7,Glu22,Arg26,Arg34, Lys37]GLP-1-(7-37)amide; DesaminoHis7,Glu22,Arg26,Arg34,Lys37]GLP-1-(7-37); Aib8,Glu22,Arg26,Lys31] GLP-1-(7-37); Aib8,Lys20,Glu22,Arg26,Glu30,Pro37]GLP-1-(7-37)amide; and DesaminoHis7,Glu22,Arg26,Arg34, Lys37] GLP-1-(7-37)..

36. The pharmaceutical composition according to embodiment 32, wherein said insulinotropic peptide is selected from the group consisting of:

N-epsilon26--(17-carboxyheptadecanoyl)-[Aib8,Arg34]GLP-1-(7-37)-peptide,
N-epsilon26-(19-carboxynonadecanoyl)-[Aib8,Arg34]GLP-1-(7-37)-peptide,
N-epsilon26-(4-{[N-(2-carboxyethyl)-N-(15-carboxypentadecanoyl)amino]methyl} benzoyl)[Arg34]GLP-1-(7-37),
N-epsilon26-[2-(2-[2-(2-[2-(2-[4-(17-Carboxyheptadecanoylamino)-4(S)-carboxybutyrylamino] ethoxy)ethoxy]acetylamino)ethoxy]ethoxy)acetyl][Aib8,Arg34]GLP-1-(7-37)peptide, N-epsilon37{2-[2-(2-{2-[2-((R)-3-carboxy-3-{[1-(19-carboxynonadecanoyl)piperidine-4-carbonyl]amino}propionylamino)ethoxy]ethoxy} acetylamino)ethoxy]ethoxy}acetyl [desaminoHis7,Glu22,Arg26,Arg34,Lys37]GLP-1(7-37)amide,
N-epsilon37{2-[2-(2-{2-[2-((S)-3-carboxy-3-{[1-(19-carboxynonadecanoyl)piperidine-4-carbonyl]amino}propionylamino)ethoxy]ethoxy}acetylamino)ethoxy]ethoxy} acetyl Aib8,Glu22,Arg26,Arg34,Lys37]GLP-1-(7-37) amide,
N-epsilon37-[2-(2-[2-(2-[2-(2-((R)-3-[1-(17-Carboxyheptadecanoyl)piperidin-4-ylcarbonylamino]3- carboxypropionylamino)ethoxy)ethoxy]acetylamino) ethoxy]ethoxy)acetyl][DesaminoHis7, Glu22 Arg26, Arg 34, Phe(m-CF3)28]GLP-1-(7-37)amide,
N-epsilon30{2-[2-(2-{2-[2-((S)-3-carboxy-3-{[1-(19-carboxynonadecanoyl)piperidine-4-carbonyl]amino}propionylamino)ethoxy]ethoxy}acetylamino)ethoxy]ethoxy} acetyl [Aib8,Glu22,Arg26,Lys30]GLP-1-(7-37), N-epsilon31{2-[2-(2-{2-[2-((S)-3-carboxy-3-{[1-(19-carboxynonadecanoyl)piperidine-4-carbonyl]amino}propionylamino)ethoxy]ethoxy}acetylamino)ethoxy]ethoxy} acetyl [Aib8, Glu22, Arg26,Lys 31]GLP-1-(7-37),
N-epsilon31-(2-{2-[2-(2-{2-[2-((S)-3-Carboxy-3-{[1-(19-carboxy-nonadecanoyl)piperidine-4-carbonyl]amino} propionylamino)ethoxy]ethoxy}acetylamino)ethoxy]ethoxy}acetyl) [Aib8,Glu22,Arg26,Lys31,Arg34]GLP-1-(7-37),
N-epsilon37-[2-(2-{2-[2-(2-{2-[(S)-4-Carboxy-4-({trans-4-[(19-carboxy-nonadecanoyl-amino)methyl]cyclohexanecarbonyl}amino)butyrylamino]ethoxy}ethoxy)acetylamino] ethoxy}ethoxy)acetyl][Aib8,Glu22,Arg26,Arg34, Lys37]GLP-1-(7-37)amide, N-epsilon37-[2-(2-{2-[2-(2-{2-[(S)-4-Carboxy-4-({trans-4-[(19-carboxy-nonadecanoylamino)methyl]cyclohexanecarbonyl}amino)butyrylamino]ethoxy}ethoxy) acetylamino]ethoxylethoxy) acetyl][DesaminoHis7,Glu22,Arg26,Arg34,Lys37]GLP-1-(7-37)amide, N-epsilon37-[2-(2-{2-[2-(2-{2-[(S)-4-Carboxy-4-({trans-4-[(19-carboxy-nonadecanoyl-amino)methyl]cyclohexanecarbonyl}amino)butyrylamino] ethoxy}ethoxy)acetylamino] ethoxy}ethoxy)acetyl][DesaminoHis7,Glu22,Arg26,Arg34,Lys37]GLP-1-(7-37), N-epsilon37-[2-(2-{2-[2-(2-{2-[(S)-4-Carboxy-4-({trans-4-[(19-carboxy-nonadecanoylamino)methyl]cyclohexanecarbonyl}amino)butyrylamino] ethoxylethoxy)acetylamino]ethoxylethoxy)acetyl][DesaminoHis7,Glu22,Arg26, Glu30,Arg34, Lys37]GLP-1-(7-37), N-epsilon20-[2-(2-{2-[(S)-4-Carboxy-4-((S)-4-carboxy-4-{12-[4-(16-(1H-tetrazol-5-yl)hexadecanoylsulfamoyl)butyrylamino]dodecanoylamino}butyrylamino)butyrylamino] ethoxy} ethoxy)acetyl][Aib8,Lys20,Glu22,Arg26,Glu30,Pro37]GLP-1-(7-37)amide, N-epsilon37-[2-(2-{2-[(S)-4-Carboxy-4-((S)-4-carboxy-4-{12-[4-(16-(1H-tetrazol-5-yl)hexadecanoylsulfamoyl)butyrylamino]dodecanoylamino} butyrylamino)butyrylamino] ethoxy}ethoxy)acetyl][Aib8,Glu22,Arg26,Arg34,Lys37]GLP-1-(7-37)amide, N-epsilon37-[2-(2-{2-[(S)-4-Carboxy-4-((S)-4-carboxy-4-{12-[4-(16-(1H-tetrazol-5-yl)hexadecanoylsulfamoyl)butyrylamino]dodecanoylamino}butyrylamino)butyrylamino] ethoxy}ethoxy)acetyl][DesaminoHis7,Glu22,Arg26, Arg34,Lys37]GLP-1-(7-37)amide, [Aib8,Glu22,Arg26,Glu30,Pro37]GLP-1-(7-37)Lys [2-(2-{2-[4-Carboxy-4-(4-carboxy-4-{4-[4-(16-1H-tetrazol-5-yl-hexadecanoylsulfamoyl)butyrylamino]butyrylamino} butyrylamino)butyrylamino]ethoxy}ethoxy)acetyl],
N-epsilon37 (Polyethyleneglycol2000)[DesaminoHis7,Glu22,Arg26,Arg34,Lys37]GLP-1 (7-37) amide,
N-epsilon37 (3-((2-(2-(2-(2-(2-Hexadecyloxyethoxy)ethoxy)ethoxy)ethoxy)ethoxy)) propionyl) [DesaminoHis7,Glu22,Arg26,Arg34,Lys37]GLP-1(7-37)-amide, N-epsilon37-{2-(2-(2-(2-[2-(2-(4-(hexadecanoylamino)-4-carboxybutyrylamino)ethoxy) ethoxy]acetyl)ethoxy)ethoxy)acetyl)}-[desaminoHis7,Glu22,Arg26, Glu30,Arg34, Lys37] (GLP-1-(7-37)amide, N-epsilon37-{2-(2-(2-(2-[2-(2-(4-(hexadecanoylamino)-4-carboxybutyrylamino) ethoxy) ethoxy]acetyl)ethoxy)ethoxy)acetyl)}-[desaminoHis7,Glu22, Arg26,Arg34,Lys 37] (GLP-1-(7-37)amide, N-epsilon37-(2-(2-(2-(2-(2-(2-(2-(2-(2-Octadecanoylamino)ethoxy)ethoxy)acetylamino) ethoxy)ethoxy) acetylamino)ethoxy)ethoxy)acetyl) [desaminoHis7,Glu22,Arg26,Arg34,Lys37] GLP-1 (7-37)amide, N-epsilon36-(2-(2-(2-((2-[2-(2-(17-carboxyheptadecanoylamino)ethoxy)ethoxy] acetylamino)ethoxy)ethoxy) acetyl) [Aib8,Glu22,Arg26,Glu30,Lys36] GLP-1-(7-37)Glu-amide,
N-epsilon37-[4-(16-(1H-Tetrazol-5-yl)hexadecanoylsulfamoyl)butyryl] [DesaminoHis7,Glu22,Arg26,Arg34,Lys37]GLP-1-(7-37)amide,
N-epsilon37-[2-(2-{2-[2-(2-{2-[(S)-4-Carboxy-4-(19-carboxynonadecanoyl amino)butyrylamino]ethoxy}ethoxy) acetylamino]ethoxy}ethoxy)acetyl][DesaminoHis7 ,Glu22,Arg26,Arg34,Lys37]GLP-1-(7-37), and N-epsilon31-[2-(2-{2-[2-(2-{2-[4-Carboxy-4-(17-carboxy-heptadecanoylamino) butyrylamino]ethoxy}ethoxy)acetylamino]ethoxy}ethoxy)acetyl][Aib8,Glu22,Arg26,Ly s31]GLP-1-(7-37).

37. The pharmaceutical composition according to embodiment 32, wherein said insulinotropic peptide is Arg34, Lys26(Nε-(γ-Glu(Nα-hexadecanoyl)))-GLP-1(7-37).

38. The pharmaceutical composition according to any one of the precedingembodiments, wherein the concentration of said insulinotropic peptide is in the range from about 0.1 mg/ml to about 25 mg/ml, in the range from about 1 mg/ml to about 25 mg/ml, in the range from about 2 mg/ml to about 15 mg/ml, in the range from about 3 mg/ml to about 10 mg/ml, or in the range from about 5 mg/ml to about 8 mg/ml.

39. The pharmaceutical composition according to embodiment 32, wherein said insulinotropic peptide is exendin-4 or ZP-10, i.e. HGEGTFTSDLSKQMEEEAVRLFIEWLKNGGPSSGAPPSKKKKKK-NH2.

40. The pharmaceutical composition according to any of embodiments 1-32, wherein said insulinotropic peptide is acylated exendin-4 or an acylated exendin-4 analogue.

41. The pharmaceutical composition according to embodiment32, wherein said insulinotropic peptide is [N-epsilon (17-carboxyheptadecanoic acid)Lys20 exendin-4(1-39)-amide

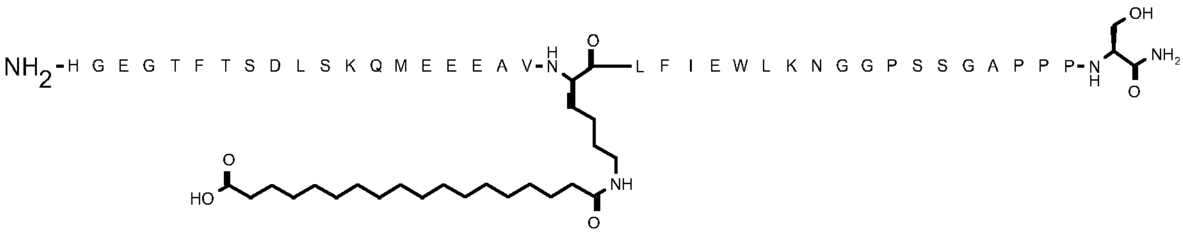

or N-epsilon32-(17-carboxy-heptadecanoyl)[Lys32]exendin-4(1-39)amide

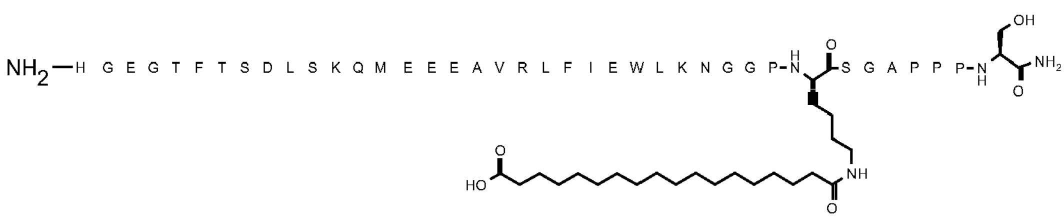

42. The pharmaceutical composition according to any one of embodiments 39-41, wherein the concentration of said

insulinotropic peptide in the pharmaceutical composition is from about 5μg/mL to about 10mg/mL, from about 5μg/mL to about 5mg/mL, from about 5μg/mL to about 5mg/mL, from about 0.1mg/mL to about 3mg/mL, or from about 0.2mg/mL to about 1mg/mL.

43. The pharmaceutical composition according to any one of embodiments 1-42, wherein the composition is adapted for pulmonary treatment, oral treatment, nasal treatment or buccal treatment.

44. The pharmaceutical composition according to any one of embodiments 1-42, wherein the composition is adapted for pulmonary use.

45. The pharmaceutical composition according to any one of embodiments 1-42, wherein the composition is adapted for nasal treatment.

46. The pharmaceutical composition according to any one of embodiments 1-42, wherein the composition is adapted for oral use.

47. The pharmaceutical composition according to any one of embodiments 1-42, wherein the composition is adapted for buccal use.

48. A method of treating diabetes in a patient in need of such a treatment, comprising administering to the patient a therapeutically effective amount of a pharmaceutical composition according to any one of the embodiments 1-47.

49. A method of treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, CNS disorders such as Alzheimer's, stroke, inflammatory bowel syndrome, dyspepsia and gastric ulcersin a patient in need of such a treatment, comprising administering to the patient a therapeutically effective amount of a pharmaceutical composition according to any one of the embodiments 1-47.

50. A method of delaying or preventing disease progression in type 2 diabetesin a patient in need of such a treatment, comprising administering to the patient a therapeutically effective amount of a pharmaceutical composition according to any one of the embodiments 1-47.

51. Pharmaceutical composition according to any one of embodiments 1-47for use as a medicament for treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, CNS disorders such as Alzheimer's, stroke, inflammatory bowel syndrome, dyspepsia and gastric ulcers.

52. Pharmaceutical composition according to any one of embodiments 1-47for use as a medicament for delaying or preventing disease progression in type 2 diabetes.

The following is a list of aspects further describing the invention:

1a. Pharmaceutical non-aqueous composition comprising a mixture of

a) a dehydrated therapeutically active insulinotropic peptide, and

b) at least onesemi-polar protic organic solvent

which insulinotropic peptide has been dehydratedat a target pH which is at least 1 pH unit from the pI of the insulinotropic peptide in aqueous solution.

2a. The pharmaceutical composition according to aspect 1a, wherein the organic solvent is selected from the group consisting of propylene glycol and glycerol.

3a. The pharmaceutical composition according to any one of aspects 1a-2a, wherein the solubility of dehydrated insulinotropic peptide in the organic solvent is at least 20 mg/ml.

4a. The pharmaceutical composition according to any one of aspects 1a-3a, wherein thetarget pH is in the range from about 6.0 to about 9.0.

5a. The pharmaceutical composition according to any one of aspects 1a-4a, wherein the organic solvent is present in an amount of at least 20% w/w.

6a. The pharmaceutical composition according to any one of aspects 1a-5a, which comprises less than 10% w/w water.

7a. The pharmaceutical composition according to any one of aspects 1a-6a, wherein the composition is adapted for pulmonary treatment, oral treatment, nasal treatment or buccal treatment.

8a. The pharmaceutical composition according to any one of aspects 1a-7a, wherein the insulinotropic peptide is selected from the group consisting ofGLP-1, GLP-2, Exendin-3, Exendin-4 and analogues and derivatives thereof.

9a. The pharmaceutical composition according to any one of aspects 1a-8a, wherein the insulinotropic peptide is a DPP-IV protected peptide.

10a. Pharmaceutical composition according to any one of aspects 1a-9a for use as a medicament for treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, CNS disorders such as Alzheimer's, stroke, inflammatory bowel syndrome, dyspepsia and gastric ulcers.

All references, including publications, patent applications, and patents, cited herein are hereby incorporated by reference in their entirety and to the same extent as if each reference were individually and specifically indicated to be incorporated by reference and were set forth in its entirety herein (to the maximum extent permitted by law).

All headings and sub-headings are used herein for convenience only and should not be construed as limiting the invention in any way.

The use of any and all examples, or exemplary language (e.g., "such as") provided herein, is intended merely to better illuminate the invention and does not pose a limitation on the scope of the invention unless otherwise claimed.No language in the specification should be construed as indicating any non-claimed element as essential to the practice of the invention.

The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

## EXAMPLES

General procedure

Thioflavin T (ThT) fibrillation assay: Principle and examples

Low physical stability of a peptide may lead to amyloid fibril formation, which is observed as well-ordered, thread-like macromolecular structures in the sample eventually resulting in gel formation. This has traditionally been measured by visual inspection of the sample. However, that kind of measurement is very subjective and depending on the observer. Therefore, the application of a small molecule indicator probe is much more advantageous. Thioflavin T (ThT) is such a probe and has a distinct fluorescence signature when binding to fibrils [Naiki et al. (1989) Anal. Biochem. 177, 244-249; LeVine (1999) Methods. Enzymol. 309, 274-284].

Dynamic (accelerated) ThT assay

The time course for fibril formation can be described by a sigmoidal curve with the following expression [Nielsen et al. (2001) Biochemistry 40, 6036-6046], cn.f figure 6:

$$F = f_i + m_i t + \frac{f_f + m_f t}{1 + e^{-[(t - t_0)/\tau]}} \quad \text{Eq.(1)}$$

Here, F is the ThT fluorescence at the time t. The constant t0 is the time needed to reach 50% of maximum fluorescence. The two important parameters describing fibril formation are the lag-time calculated by t0 - 2τ and the apparent rate constant kapp = 1/τ.

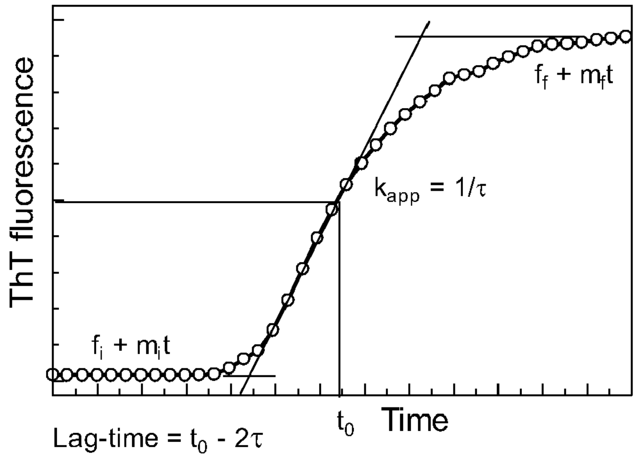


Formation of a partially folded intermediate of the peptide is suggested as a general initiating mechanism for fibrillation. Few of those intermediates nucleate to form a template onto which further intermediates may assembly and the fibrillation proceeds. The lag-time corresponds to the interval in which the critical mass of nucleus is built up and the apparent rate constant is the rate with which the fibril itself is formed.

Sample preparation

Samples were prepared freshly before each assay. Thioflavin T was added to the samples from a stock solution in $H_2O$ to a final concentration of 1 μM. Sample aliquots of 200 μl were placed in a 96 well microtiter plate (Packard OptiPlate™-96, white polystyrene). Usually, eight replica of each sample (corresponding to one test condition) were placed in one column of wells. The plate was sealed with Scotch Pad (Qiagen).

Incubation and fluorescence measurement

Incubation at given temperature, shaking and measurement of the ThT fluorescence emission were done in a FluoroskanAscentFL fluorescence platereader (Thermo Labsystems). The temperature was adjusted to 37 °C. The orbital shaking was adjusted to 960 rpm with an amplitude of 1 mm in all the presented data. Fluorescence measurement was done using excitation through a 444 nm filter and measurement of emission through a 485 nm filter.

Each run was initiated by incubating the plate at the assay temperature for 10 min. The plate was measured every 20 minutes for typically 45 hours. Between each measurement, the plate was shaken and heated as described.

Static ThT assay

Sample preparation and fluorescence measurement

Samples containing insulin aspart in non-aqueous propylene glycol was stored at 5 and 40°C for up to 1 month. The samples were prior to measurement diluted to an expected concentration (including fibrillated and native protein) of 1 mg/ml with demineralised water. Fluorescence measurements were performed in semimicro quartz cuvettes (Hellma, Germany) with a 0.3 cm excitation light path using a Perkin-Elmer model LS 50 B luminescence spectrometer. Emission spectra from 470 to 560 nm with excitation at 450 nm and with slit widths of 5 nm were recorded immediately after addition of 4 μl ThT 1 mM stock solution to 196 μl diluted sample. The concentration of ThT in reaction mixture was 20 μMol/l. The results were reported as I 482 nm /[protein concentration (mg/ml) x cuvette path length (cm)], i.e. the results were normalized to a protein concentration of 1 mg/ml and a cuvette path length of 1 cm.

EXAMPLE 1

pMDI solution type formulation of Arg34, Lys26(Nε-(γ-Glu(Nα-hexadecanoyl)))-GLP-1(7-37).

The composition was the following:

| | |
|---|---|
| 1,2 Propanediol (propylene glycol)with 3.5 mg Arg34, Lys26(Nε-(γ-Glu(Nα-hexadecanoyl)))-GLP-1(7-37) per ml | 19 % |
| Ethanol 96% | 30 % |
| HFA 134a | 50 % |
| Water | 1% |

Arg34, Lys26(Nε-(γ-Glu(Nα-hexadecanoyl)))-GLP-1(7-37), with a pI of 5.6 and a target pH of of 8.7,was dissolved into the polar co-solvents (propylene glycol, ethanol and water) and filled into a 14 ml glass canister. The canister was closed with a spray valve (Valois, Le Vaudreuil, France) and HFA propellant was filled into the canister with a Pamasol 2005/10 filling machine.

The formulation was slightly cloudy, which might be due to trace amounts of insoluble inorganic salts.

The particle size distribution was evaluated by laser diffraction analysis (Sympatec GmbH, Germany) equipped with Windox 4 software.

EXAMPLE 2

Solubility of an insulinotropic peptide in propylene glycol or glycerol at ambient temperature

Increasing amounts of N-epsilon20-{2-(2-(2-(2-[2-(2-(4-(hexadecanoylamino)-4-carboxybutyrylamino)ethoxy) ethoxy]acetylamino)ethoxy)ethoxy)acetyl)}-[Leu14,Lys20,Gln28]-Exendin-4,with a pI of 4.2 and a target pH of 7.4, were added to 0.5 ml of propylene glycol and glycerol, respectively. The solvents were gently agitated and inspected for clarity of each addition of compound. The solubility limit was not reached after addition of 211 mg compound to 0.5 ml of propylene glycol and 98 mg of compound to glycerol, although the solutions were very viscous.

EXAMPLE 3.

Formulation of a non-aqueous solution type pressurized metered dose-inhaler with a insulinotropic peptide

N- epsilon20- {2-(2-(2-(2-[2-(2-(4-(hexadecanoylamino)- 4- carboxybutyrylamino) ethoxy) ethoxy] acetylamino) ethoxy)ethoxy)acetyl)}-[Leu14,Lys20,Gln28]-Exendin-4,with a pI of 4.2 and a target pH of 7.4, was dissolved in the solvent (mixture of ethanol and propanol as listed in Table 2) and pipetted into an aerosol glass container, crimped with a standard valve (Valois DF10-50) and pressurized with the corresponding portion of liquefied propellant. Crimping and filling was done with Pamasol 2005/10 filling system. The vials were inspected for clarity.

Formulations F1-6 did not show any precipitation of the active immediately after filling; however, all investigated compositions finally showed some kind of precipitate after storage for a few days or after mechanical stress (shaking). The most promising formulation was found to be F6 containing 17.5% ethanol, 12.5% propylene glycol and 67.5% HFA 227. This formulation did not show precipitation until day 21 after manufacture. However, after 3weeks storage a slight precipitate was observed which was easily redispersable.

Table 2: Formulations showing least precipitation and no / minimal glass container adsorption

| | F1 | F2 | F3 | F4 | F5 | F6 |
|---|---|---|---|---|---|---|
| Conc. of API | 10 µg /shot | 10 µg / shot | 10 µg / shot | 10 µg / shot | 10 µg / shot | 10 µg / shot |

(continued)

| | F1 | F2 | F3 | F4 | F5 | F6 |
|---|---|---|---|---|---|---|
| Propylene glycol [%] | 12.5 | 12.5 | 10 | 17.5 | 10 | 12.5 |
| Ethanol 96% [%] | 15 | 20 | 20 | 12.5 | 20 | 17.5 |
| HFA 134a [%] | 72.5 | 67.5 | 70 | 70 | - | - |
| HFA 227 [%] | - | - | - | - | 70 | 70 |
| | 100.0 | 100.0 | 100.0 | 100.0 | 100.0 | 100.0 |

For particle spray size analysis, formulation F6 was freshly prepared and sprayed for aerosol droplet size measurement using different actuator orifice diameters.

The droplet size distributions of formulation F6 were determined by laser diffraction (HELOS-laser diffractometer, SympaTEC GmbH, Germany).

The MDI was positioned in front of the laser beam at a distance of approx. 12 cm. The measurements were performed when the optical concentration of aerosol was > 0.1. The R3 lens (measuring range from 0.5 μm to 175 μm) was used. The raw data for the determination of particle size distribution was evaluated by the Sympatec Windox 5 Software according to the Fraunhofer theory.

Formulation F6 with 12.5% propylene glycol and 17.5% ethanol showed average particles sizes of roughly 25 μm and and a d90% of roughly 40 μm. A trend towards lower volume mean diameters could be seen when a smaller actuator orifice diameter was used. The size distribution was compared with an HFA solution formulation of Budesonide (Budes N®, Sandoz) which was found to deliver an average diameter of 8 μm and a d90% of approx. 15 μm.

## Claims

**1.** Pharmaceutical non-aqueous composition comprising a mixture of

a) a dehydrated therapeutically active insulinotropic peptide, and
b) at least onesemi-polar protic organic solvent
whichinsulinotropic peptide has been dehydratedat a target pH which is at least 1 pH unit from the pI of the insulinotropic peptide in aqueous solution.

2. The pharmaceutical composition according to claim 1, wherein the organic solvent is selected from the group consisting of propylene glycol and glycerol.

3. The pharmaceutical composition according to any one of claims 1-2, wherein the solubility of dehydrated insulinotropic peptide in the organic solvent is at least 20 mg/ml.

4. The pharmaceutical composition according to any one of claims 1-3, wherein thetarget pH is in the range from about 6.0 to about 9.0.

5. The pharmaceutical composition according to any one of claims 1-4, wherein the organic solvent is present in an amount of at least 20% w/w.

6. The pharmaceutical composition according to any one of claims 1-5, which comprises less than 10% w/w water.

7. The pharmaceuticalcompositionaccording to anyone of claims 1-28, wherein the composition is for pulmonary, parenteral, nasal or oral treatmentof diabetes or hyperglycaemia.

8. The pharmaceutical composition according to any one of claims 1-7, wherein the insulinotropic peptide is selected from the group consisting ofGLP-1, GLP-2, Exendin-3, Exendin-4 and analogues and derivatives thereof.

9. The pharmaceutical composition according to any one of claims 1-8, wherein the insulinotropic peptide is a DPP-IV protected peptide.

10. Pharmaceutical composition according to any one of claims 1-9 for use as a medicament for treatment or prevention of hyperglycemia, type 2 diabetes, impaired glucose tolerance, type 1 diabetes, obesity, hypertension, syndrome X, dyslipidemia, cognitive disorders, atheroschlerosis, myocardial infarction, coronary heart disease and other cardiovascular disorders, CNS disorders such as Alzheimer's, stroke, inflammatory bowel syndrome, dyspepsia and gastric ulcers.

11. A process for the preparation of a pharmaceutical solution by:

a) providing an aqueous solution of a therapeutically active insulinotropic peptide optionally comprising excipients,
b) adjusting the pH value to a target pH value which is 1 unit, preferably 2 units and more preferably 2.5 pH units above or below the pI of the insulinotropic peptide,
c) removing water (dehydrating) the insulinotropic peptide by conventional drying technologies such as freeze- and spray drying, and
d) mixing and dissolution of the insulinotropic peptide in a semi-polar protic non-aqueous solvent e.g. by stirring, tumbling or other mixing methods,
e) optionally filtration or centrifugation of the non-aqueous insulinotropic peptide solution to remove non-dissolved inorganic salts,
f) optionally removing residual amounts of waters by e.g. adding solid dessicants or vacuum drying,
g) optionallyaddingfurtherexcipientssuch as a hydrofluoroalkanepropellants and cosolvents for solution pressurizedmetereddoseinhalers or adding detergents, polymers, lipids and co-solvents for oral dosage forms.

12. A dehydrated pharmaceutically active polypeptide obtainable by the process of dehydrating the polypeptide at a target pH which is at least 1 pH unit from the pI of the polypeptide in aqueous solution.

13. The dehydrated pharmaceutically active polypeptide according to claim 12, wherein the solubility of said polypeptide is at least 20 mg/mL in organic solvent

14. The dehydrated pharmaceutically active polypeptide according to claim 13, wherein said organic solvent is a semi-polar protic organic solvent.

15. The dehydrated pharmaceutically active polypeptide according to any one of the claims 12-14, wherein said organic solvent is a polyol.

Europäisches Patentamt
European Patent Office
Office européen des brevets

# EUROPEAN SEARCH REPORT

Application Number
EP 12 15 9415

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/035775 A1 (KLIBANOV ALEXANDER M [US]) 20 February 2003 (2003-02-20)<br>* paragraphs [0011], [0012], [0030], [0037]; claims 1,3,6,7 *<br>----- | 1-15 | INV.<br>A61K9/00<br>A61K9/08<br>A61K9/19<br>A61K38/04<br>A61K38/16<br>A61K47/10<br>A61K47/42<br>A61K9/12<br>A61K38/28 |
| X | WO 2004/105790 A (NOVO NORDISK AS [DK]; JUUL-MORTENSEN CLAUS [DK])<br>9 December 2004 (2004-12-09)<br>* page 2, line 1 - page 3, line 2; claims 1,34,36,37,41 *<br>* page 6, line 34 - page 7, line 15 *<br>----- | 1-15 | |
| A | WO 2007/047948 A (NASTECH PHARM CO [US]; QUAY STEVEN C [US]; COHEN ANNEMARIE STOUDT [US]) 26 April 2007 (2007-04-26)<br>* paragraphs [0041], [0053], [0055] - [0057], [0163] *<br>* page 7, line 11 - line 37 *<br>* page 14, line 3 - page 16, line 15 *<br>* page 53, line 14 - line 28 *<br>----- | 1-10 | |
| A | US 2005/276843 A1 (QUAY STEVEN C [US] ET AL) 15 December 2005 (2005-12-15)<br>* paragraphs [0026] - [0035], [0082] *<br>----- | 1-15 | TECHNICAL FIELDS SEARCHED (IPC)<br>A61K |
| A | EP 1 002 547 A (HOFFMANN LA ROCHE [CH])<br>24 May 2000 (2000-05-24)<br>* claims 1,4,5 *<br>----- | 1-10 | |

The present search report has been drawn up for all claims

1

| Place of search | Date of completion of the search | Examiner |
|---|---|---|
| Munich | 3 August 2012 | Giese, Hans-Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 9415

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2012

| Patent document cited in search report | Publication date | Patent family member(s) | Publication date |
|---|---|---|---|
| US 2003035775 A1 | 20-02-2003 | US 2003035775 A1 | 20-02-2003 |
| | | WO 0042993 A2 | 27-07-2000 |
| WO 2004105790 A | 09-12-2004 | EP 1633391 A1 | 15-03-2006 |
| | | JP 2007524592 A | 30-08-2007 |
| | | US 2008318865 A1 | 25-12-2008 |
| | | WO 2004105790 A1 | 09-12-2004 |
| WO 2007047948 A | 26-04-2007 | AT 471144 T | 15-07-2010 |
| | | AU 2006304757 A1 | 26-04-2007 |
| | | CA 2626357 A1 | 26-04-2007 |
| | | EP 1951198 A2 | 06-08-2008 |
| | | JP 2009512709 A | 26-03-2009 |
| | | KR 20080064171 A | 08-07-2008 |
| | | US 2010210506 A1 | 19-08-2010 |
| | | WO 2007047948 A2 | 26-04-2007 |
| US 2005276843 A1 | 15-12-2005 | AU 2005247369 A1 | 08-12-2005 |
| | | CA 2567056 A1 | 08-12-2005 |
| | | CN 101151048 A | 26-03-2008 |
| | | EP 1750756 A2 | 14-02-2007 |
| | | JP 2007537274 A | 20-12-2007 |
| | | US 2005276843 A1 | 15-12-2005 |
| | | US 2007154400 A1 | 05-07-2007 |
| | | US 2007154401 A1 | 05-07-2007 |
| | | US 2007167364 A1 | 19-07-2007 |
| | | US 2007244049 A1 | 18-10-2007 |
| | | US 2010184688 A1 | 22-07-2010 |
| | | WO 2005115441 A2 | 08-12-2005 |
| EP 1002547 A | 24-05-2000 | AR 023044 A1 | 04-09-2002 |
| | | AT 318616 T | 15-03-2006 |
| | | AU 755930 B2 | 02-01-2003 |
| | | BR 9903984 A | 13-03-2001 |
| | | CA 2281049 A1 | 01-03-2000 |
| | | CN 1250669 A | 19-04-2000 |
| | | CO 5271726 A1 | 30-04-2003 |
| | | DE 69930033 T2 | 12-10-2006 |
| | | EP 1002547 A1 | 24-05-2000 |
| | | ES 2258830 T3 | 01-09-2006 |
| | | GC 0000107 A | 29-06-2005 |
| | | HR P990272 A2 | 30-06-2000 |
| | | HU 9902952 A1 | 28-06-2000 |
| | | ID 22964 A | 23-12-1999 |
| | | JP 3664373 B2 | 22-06-2005 |
| | | JP 2000086532 A | 28-03-2000 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 12 15 9415

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2012

| Patent document cited in search report | Publication date | Patent family member(s) | Publication date |
|---|---|---|---|
| | | KR 20000022777 A | 25-04-2000 |
| | | MA 26682 A1 | 20-12-2004 |
| | | NO 994214 A | 02-03-2000 |
| | | NZ 337527 A | 22-12-2000 |
| | | PL 335203 A1 | 13-03-2000 |
| | | RU 2180855 C2 | 27-03-2002 |
| | | SG 85670 A1 | 15-01-2002 |
| | | TR 9902103 A2 | 21-04-2000 |
| | | TW 570805 B | 11-01-2004 |
| | | US 2002028766 A1 | 07-03-2002 |
| | | US 2005123610 A1 | 09-06-2005 |
| | | US 2008064854 A1 | 13-03-2008 |
| | | ZA 9905601 A | 27-09-2000 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0042993 A
- WO 03078538 A
- US 5957895 A
- US 5858001 A
- US 4468221 A
- US 6074 A
- US 5527288 A
- US 6074369 A

### Non-patent literature cited in the description

- **J. T. CHIN ; S. L. WHEELER ; A. M. KLIBANOV.** Communication to the editor: On protein solubility in organic solvents. *BIOTECHNOL.BIOENG.,* 1994, vol. 44 (1), 140-145
- **HUDSON ; ANDERSEN.** *Peptide Science,* 2004, vol. 76 (4), 298-308
- **GREENE ; WUTS.** Protective Groups in Organic Synthesis. John Wiley & Sons, 1999
- **SIEGEL et al.** *Regul. Pept.,* 1999, vol. 79, 93-102
- **MENTLEIN et al.** *Eur. J. Biochem.,* 1993, vol. 214, 829-35
- **NAIKI et al.** *Anal. Biochem.,* 1989, vol. 177, 244-249
- **LEVINE.** *Methods. Enzymol.,* 1999, vol. 309, 274-284
- **NIELSEN et al.** *Biochemistry,* 2001, vol. 40, 6036-6046

SEQUENCE LISTING

```
<110>  Novo Nordisk A/S

<120>  STABLE NON-AQUEOUS PHARMACEUTICAL COMPOSITIONS

<130>  7634.204-WO

<160>  4

<170>  PatentIn version 3.4

<210>  1
<211>  31
<212>  PRT
<213>  homo sapiens

<400>  1

His Ala Glu Gly Thr Phe Thr Ser Asp Val Ser Ser Tyr Leu Glu Gly
1               5                   10                  15


Gln Ala Ala Lys Glu Phe Ile Ala Trp Leu Val Lys Gly Arg Gly
            20                  25                  30


<210>  2
<211>  40
<212>  PRT
<213>  Heloderma suspectum


<220>
<221>  MISC_FEATURE
<222>  (40)..(40)
<223>  Xaa is NH2

<400>  2

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Pro Ser Xaa
        35                  40


<210>  3
<211>  40
<212>  PRT
<213>  Artificial

<220>
<223>  Derivative of Exendin-4


<220>
<221>  MISC_FEATURE
<222>  (20)..(20)
<223>  Xaa is N-epsilon(17-carboxyheptadecanoic acid)Lys
```

```
<220>
<221>  MISC_FEATURE
<222>  (40)..(40)
<223>  Xaa is NH2

<400>  3

His Gly Glu Gly Thr Phe Thr Ser Asp Leu Ser Lys Gln Met Glu Glu
1               5                   10                  15

Glu Ala Val Xaa Leu Phe Ile Glu Trp Leu Lys Asn Gly Gly Pro Ser
            20                  25                  30

Ser Gly Ala Pro Pro Pro Ser Xaa
        35                  40


<210>  4
<211>  40
<212>  PRT
<213>  Artificial

<220>
<223>  Derivative of Exendin-4


<220>
<221>  MISC_FEATURE
<222>  (32)..(32)
<223>  Xaa is N-epsilon-(17-carboxy-heptadecanoyl)Lys

<220>
<221>  MISC_FEATURE
<222>  (40)..(40)
<223>  Xaa is NH2

<400>  4

His Gly Glu Gly Thr Phe Thr Ser Asp Leu Ser Lys Gln Met Glu Glu
1               5                   10                  15

Glu Ala Val Arg Leu Phe Ile Glu Trp Leu Lys Asn Gly Gly Pro Xaa
            20                  25                  30

Ser Gly Ala Pro Pro Pro Ser Xaa
        35                  40
```